# EUROPEAN PATENT APPLICATION

(11) **EP 4 786 594 A1**
(43) Date of publication of application: **05.08.2026**
(21) Application number: 24870941.2
(22) Date of filing: 27.09.2024
(51) Int. Cl.: C12N 15/113, C12N 15/12, A61K 48/00, A61K 38/42, A61P 7/06

(54) **COMPOSITION AND METHOD FOR TREATING HEMOGLOBIN DISEASES**

(30) Priority: 28.09.2023 CN 202311287074
(71) Applicant: Shanghai Vitalgen Biopharma Co., Ltd., Shanghai 201210 (CN)
(72) Inventor: QU, Su, Shanghai 201210 (CN); ZHAO, Yuemeng, Shanghai 201210 (CN); TIAN, Shin-shay, Shanghai 201210 (CN); ZHAO, Xiaoping, Shanghai 201210 (CN)
(74) Representative: Weickmann & Weickmann PartmbB
(86) International application number: PCT/CN2024/121666
(87) International publication number: WO 2025/067403

(57) **Abstract**

Provided are a gRNA molecule and a nucleic acid, complex, composition, cell, and kit thereof. Also provided is a method for increasing the HbF expression level in progeny red blood cells of human CD34+ HSPCs by means of genome editing, the method can be used for treating hemoglobinopathies such as β-thalassemia and sickle cell disease.

## Description

### Technical Field

The present application relates to the field of biomedicine, and in particular to a molecule for treating a hemoglobinopathy, a composition comprising the molecule, and a method and use thereof.

### Background Art

Hemoglobinopathies are a highly heterogeneous class of hereditary anaemias. They can be classified based on the deficient globin chain into α-hemoglobinopathies and β-hemoglobinopathies, and also include quantitative abnormalities of hemoglobin. The β-thalassemia is a heterogeneous autosomal recessive hereditary anemia characterized by reduced or absent synthesis of the β-globin chain, leading to diseases associated with hemoglobin A (HbA). Excess hemoglobin A chains can lead to dyserythropoiesis, intramedullary apoptosis of erythroid precursors, and hemolytic anemia. The β-thalassemia is clinically divided into two types, distinguished according to the severity of symptoms: β-thalassemia major (also known as β0, in which case the production of β-globin chains is halted due to mutations) and β-thalassemia intermedia (also known as β+, in which case the production of β-globin chains is reduced). The β-thalassemia major is a serious internal medical disease that requires routine blood transfusions. Sickle cell disease (SCD) is also a hereditary blood disease caused by defective β-globin chain production, which includes sickle cell anemia, sickle hemoglobin C disease (HbSC), sickle β+ thalassemia (HbS/β+), and sickle β0 thalassemia (HbS/β0).

Studies have shown that all forms of β-hemoglobinopathies are caused by mutations in the β-globin structural gene (HBB). The β-thalassemia is caused by more than 200 different mutations in the β-globin gene. SCD is caused by an A-to-T point mutation in the sixth codon that results in an E6V substitution leading to defective β-globin (βs). The human β-globin locus consists of five genes located within the short region of chromosome 11 and responsible for the production of the β-globin chain of HAb. The locus contains δ, γ-A (HBG1), γ-G (HBG2), and ε globin genes in addition to the β-globin gene. A 16-kb locus control region (LCR) is located about 40-60 kb upstream of the HBB locus and is thought to regulate the differential expression of the β-like globin genes throughout development. In the late neonatal period, the expression of γ-A and γ-G in the fetus is inhibited, while the expression of adult β-globin gene is initiated; and this process is known as γ-to-β globin expression switching. Hereditary persistence of fetal hemoglobin (HbF) (HPFH) is a benign condition characterized by sustained high-level production of HbF in adulthood. Patients heterozygous for HPFH and β-hemoglobinopathy have mild clinical symptoms, while patients homozygous for these diseases have severe symptoms. HPFH is usually caused by mutations in the β-globin gene cluster or in the γ-globin promoter region that either create new binding sites for erythroid activators or disrupt binding sites for repressors. Point mutations in the proximal promoters of HBG1 and HBG2 fall into two distinct groups, located about 115 bp and 200 bp upstream of the transcription start site (TSS) of the duplicated γ-globin genes, respectively. The repressor that plays a major role in γ-globin gene silencing is BCL11A which binds at -115 bp. Recently, ZBTB7A, also known as LRF or FBI-1, was identified as a second major repressor that binds to the site at -200bp. These findings suggest that introducing artificial HFPH-like mutations into the promoter region to reactivate γ-globin gene expression is a promising therapeutic strategy for β-hemoglobinopathies.

In China, although improved clinical treatment has reduced mortality in pediatric patients, treatment for most patients with β-hemoglobinopathies is still mainly supportive treatment. Current therapeutic approaches aim to alleviate symptoms and treat complications, including regular blood transfusions, suppression of erythropoiesis, iron chelation, and relieving pain. Human leucocyte antigen (HLA)-identical sibling bone marrow transplantation (BMT) or HLA-matched donor cord blood transplantation (CBT) is another feasible therapeutic approach, but carries the risk of graft-versus-host disease (GVHD). However, allogeneic BMT is only suitable for a small proportion of patients with β-hemoglobinopathies, because most patients can not find a fully matched unrelated donor.

Novel therapeutic approaches utilizing autologous transgenic HSPCs can avoid the need for fully matched bone marrow or cord blood donors, thereby avoiding the risk of GVHD after transplantation. Genome-wide association studies have shown that some single nucleotide polymorphisms (SNPs) located in the erythroid-specific enhancer of the BCL11A locus are related to downregulated BCL11a expression, persistent expression of γ-globin in adulthood, and clinical manifestations of mild β-hemoglobinopathy. Gene therapy using corrected β-globin has also been suggested as a possible cure for β-hemoglobinopathies. Lentivirus vectors (LVs) without HIV-1 replication elements can mediate the transfer of exogenous gene fragments into quiescent hematopoietic stem cells. The gene therapy involving autologous transplantation of HSPCs transduced with the BB305 lentivirus vector encoding the fully functional βA-T87Q-globin (ZYNTEGLO^{®} - betibeglogene autotemcel) has enabled patients with severe β-hemoglobinopathies to become free of regular blood transfusions. However, the safety of the above-mentioned gene therapy is a concern, as the random integration of the vector gene from the LV vector into the genome, along with the introduction of strong promoter and enhancer elements, may lead to activation of proto-oncogenes or other harmful events triggered by such effect.

Overall, despite recent advances in the development of genetic approaches to treat β-hemoglobinopathies, there are still unmet clinical needs for providing safe and effective treatment for these genetic diseases.

### Summary of the Invention

In order to overcome the limitations of the prior art in the treatment of hemoglobinopathies, the present application provides methods for increasing the level of HbF in human erythroid progeny of human CD34+ HSPCs by genome editing. The two γ-globin chains of HbF are expressed by HBG1 and HBG2. The molecules, compositions, cells (including autologous CD34+ HSPCs that can be infused into patients with severe hemoglobinopathies), kits, methods and the like of the present application act on the transcriptional regulatory regions of HBG1 and HBG2 and are useful in the treatment of hemoglobinopathies such as β-thalassemia and sickle cell disease.

In one aspect, the present application provides a guide RNA (gRNA) molecule, wherein the gRNA molecule comprises a targeting domain complementary to a target sequence, and the target sequence is located within an HBG1 promoter region located between chr11: 5,248,269 and 5,249,857 of human genome hg19, or within an HBG2 promoter region located between chr11: 5,253,188 and 5,254,781 of human genome hg19.

In some embodiments, the target sequence is located between about 210 bp upstream and about 100 bp upstream of transcription initiation sites of the HBG1 and HBG2.

In some embodiments, the gRNA comprises a tracr sequence and a crRNA sequence.

In some embodiments, the gRNA is a dual guide RNA molecule.

In some embodiments, the gRNA is a single guide RNA molecule (sgRNA).

In some embodiments, (1) a CRISPR/Cas system comprising the gRNA molecule is contacted with the target sequence, or (2) a CRISPR/Cas system comprising the gRNA molecule is introduced into a cell in which a gene comprising the target sequence is located, thereby enabling a base insertion or deletion (indel) to be formed at or near the target sequence.

In some embodiments, the CRISPR/Cas system comprises a Cas12b protein and/or a functionally active fragment thereof.

In some embodiments, the CRISPR/Cas system comprises a Cas12i protein and/or a functionally active fragment thereof.

In some embodiments, the N-terminus and/or C-terminus of the nuclease is linked to one or more nuclear localization signals.

In some embodiments, the nuclear localization signal is linked to a His tag.

In some embodiments, the tracr sequence comprises a nucleotide sequence set forth in SEQ ID NO: 60. For example, the tracr sequence comprises a sequence as shown below (m represents a 2-O-methyl modification, and * represents a 3**'** phosphorothioate modification):

mG*mG*mT*CGTCTATAGGACGGCGAGTTTTTCAACGGGTGTGCCAATGGCCAC
TTTCCAGGTGGCAAAGCCCGTTGAACTTCTCAAAAAGAACGCTCGCTCAGTGTTCT*
mG*mA*mC

In some embodiments, the crRNA sequence comprises a nucleotide sequence selected from any one of SEQ ID NOs: 61-66. For example, the crRNA sequence comprises a sequence as shown in the following table:

| **No.** | **Types** | **Nucleotide sequences (m represents a 2-O-methyl modification, and * represents a 3' phosphorothioate modification)** |
|---|---|---|
| 12b-crRNA1-18 | RNA | mG*mT*mC*GGATCACTGAGCGAGCGATCTGAGAAGTGGCACAGATAGT GTGGGAA*mG*mG*mG |
| 12b-crRNA1-20 | RNA | mG*mT*mC*GGATCACTGAGCGAGCGATCTGAGAAGTGGCACAGATAGT GTGGGGAAGG*mG*mG*mC |
| 12b-crRNA1-23 | RNA | mG*mT*mC*GGATCACTGAGCGAGCGATCTGAGAAGTGGCACAGATAGT GTGGGGAAGGGGC*mC*mC*mC |
| 12b-crRNA1-25 | RNA | mG*mT*mC*GGATCACTGAGCGAGCGATCTGAGAAGTGGCACAGATAGT GTGGGGAAGGGGCCC*mC*mC*mA |
| 12b-crRNA1-28 | RNA | mG*mT*mC*GGATCACTGAGCGAGCGATCTGAGAAGTGGCACAGATAGT GTGGGAAGGGGCCCCCA*mA*mG*mA |
| 12b-crRNA1-31 | RNA | mG*mT*mC*GGATCACTGAGCGAGCGATCTGAGAAGTGGCACAGATAGT GTGGGGAAGGGGCCCCCAAGA*mG*mG*mA |

In some embodiments, wherein comprises a nucleotide sequence selected from any one of SEQ ID NOs: 12-59 and 67-78. For example, the gRNA sequence comprises a sequence as shown in the following table:

| **No.** | **Types** | **Nucleotide sequences (m represents a 2-O-methyl modification, and * represents a 3' phosphorothioate modification)** |
|---|---|---|
| 12b-gRNA1-18 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAA*mG*mG*mG |
| 12b-gRNA1-20 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGG*mG*mG*mC |
| 12b-gRNA1-21 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGG*mG*mC*mC |
| 12b-gRNA1-22 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGG*mC*mC*mC |
| 12b-gRNA1-23 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGGC*mC*mC*mC |
| 12b-gRNA1-24 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGGCC*mC*mC*mC |
| 12b-gRNA1-25 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGAAGGGCCC*mC*mC*mA |
| 12b-gRNA1-26 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACAGATAGTGTGGGGAAGGGGCCCC*mC*mA*mA |
| 12b-gRNA1-27 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGGCCCCC*mA*mA*mG |
| 12b-gRNA1-28 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGGCCCCCA*mA*mG*mA |
| 12b-gRNA1-29 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGGCCCCCAA*mG*mA*mG |
| 12b-gRNA1-30 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACAGATAGTGTGGGGAAGGGGCCCCCAAG*mA*mG*mG |
| 12b-gRNA1-31 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGATAGTGTGGGGAAGGGGCCCCCAAGA*mG*mG*mA |
| 12b-gRNA2-18 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTG*mG*mG*mG |
| 12b-gRNA2-20 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGG*mG*mA*mA |
| 12b-gRNA2-21 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGGG*mA*mA*mG |
| 12b-gRNA2-22 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACCATTGAGATAGTGTGGGGA*mA*mG*mG |
| 12b-gRNA2-23 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGGGAA*mG*mG*mG |
| 12b-gRNA2-24 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGGGAAG*mG*mG*mG |
| 12b-gRNA2-25 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACCATTGAGATAGTGTGGGGAAGG*mG*mG*mC |
| 12b-gRNA2-26 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACCATTGAGATAGTGTGGGGAAGGG*mG*mC*mC |
| 12b-gRNA2-27 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGGAAGGGG*mC*mC*mC |
| 12b-gRNA2-28 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGGGAAGGGGC*mC*mC*mC |
| 12b-gRNA2-29 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACCATTGAGATAGTGTGGGGAAGGGGCC*mC*mC*mC |
| 12b-gRNA2-30 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCATTGAGATAGTGTGGGGAAGGGGCCC*mC*mC*mA |
| 12b-gRNA2-31 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACCATTGAGATAGTGTGGGGAAGGGGCCCC*mC*mA*mA |
| 12b-gRNA3-18 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGT*mG*mG*mG |
| 12b-gRNA3-20 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGG*mG*mG*mA |
| 12b-gRNA3-21 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGG*mG*mA*mA |
| 12b-gRNA3-22 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACGCATTGAGATAGTGTGGGG*mA*mA*mG |
| 12b-gRNA3-23 | RNA | mG*mC*mA*GGTCGTCTATAGGACGGCGAGTTTTTCAACGGGTGTGCCA ATGGCCACTTTCCAGGTGGCAAAGCCCGTTGAGCTTCAAAGAAGTGGC ACGCATTGAGATAGTGTGGGGA*mA*mG*mG |
| 12b-gRNA3-24 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAA*mG*mG*mG |
| 12b-gRNA3-25 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAG*mG*mG*mG |
| 12b-gRNA3-26 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAGG*mG*mG*mC |
| 12b-gRNA3-27 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAGGG*mG*mC*mC |
| 12b-gRNA3-28 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAGGGG*mC*mC*mC |
| 12b-gRNA3-29 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAGGGGC*mC*mC*mC |
| 12b-gRNA3-30 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAGGGGCC*mC*mC*mC |
| 12b-gRNA3-31 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCATTGAGATAGTGTGGGGAAGGGGCCC*mC*mC*mA |
| 12b-gRNA4 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCCAGCCTTGCCTTGAC*mC*mA*mA |
| 12b-gRNA5 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCCTTGTCAAGGCTATTG*mG*mT*mC |
| 12b-gRNA6 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGCCTTGTCAAGGCTATT*mG*mG*mT |
| 12b-gRNA7 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCAGACAGATATTTGCAT*mT*mG*mA |
| 12b-gRNA8 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACCCCACACTATCTCAATG*mC*mA*mA |
| 12b-gRNA9 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGACAGATATTTGCATT*mG*mA*mG |
| 12b-gRNA10 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACTCAAGGCTATTGGTCAA*mG*mG*mC |
| 12b-gRNA11 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACAGCAGCAGTATCCTCTT*mG*mG*mG |
| 12b-gRNA12 | RNA | mG*mC*mA*GGTCGTCTATAGGACGGCGAGTTTTCAACGGGTGTGCCA ATGGCCACTTTCCAGGTGGCAAAGCCCGTTGAGCTTCAAAGAAGTGGC ACGTCAAGTTTGCCTTGTC*mA*mA*mG |
| 12b-Aa13tracr RNA | RNA | mG*mG*mT*CGTCTATAGGACGGCGAGTTTTTCAACGGGTGTGCCAATG GCCACTTTCCAGGTGGCAAAGCCCGTTGAACTTCTCAAAAAGAACGCT CGCTCAGTGTTCT*mG*mA*mC |
| 12b-crRNA1-18 | RNA | mG*mT*mC*GGATCACTGAGCGAGCGATCTGAGAAGTGGCACAGATAG TGTGGGGAA*mG*mG*mG |
| 12b-crRNA1-20 | RNA | mG*mT*mC*GGATCACTGAGCGAGCGATCTGAGAAGTGGCACAGATAG TGTGGGAAGG*mG*mG*mC |
| 12b-crRNA1-23 | RNA | mG*mT*mC*GGATCACTGAGCGAGCGATCTGAGAAGTGGCACAGATAG TGTGGGGAAGGGGC*mC*mC*mC |
| 12b-crRNA1-25 | RNA | mG*mT*mC*GGATCACTGAGCGAGCGATCTGAGAAGTGGCACAGATAG TGTGGGGAAGGGGCCC*mC*mC*mA |
| 12b-crRNA1-28 | RNA | mG*mT*mC*GGATCACTGAGCGAGCGATCTGAGAAGTGGCACAGATAG TGTGGGAAGGGGCCCCCA*mA*mG*mA |
| 12b-crRNA1-31 | RNA | mG*mT*mC*GGATCACTGAGCGAGCGATCTGAGAAGTGGCACAGATAG TGTGGGAAGGGGCCCCCAAGA*mG*mG*mA |
| 12i-gRNA-1 | RNA | mA*mG*mA*AATCCGTCTTTCATTGACGGAGATAGTGTGGGAAGG*mG *mG*mC |
| 12i-gRNA-2 | RNA | mA*mG*mA*AATCCGTCTTTCATTGACGGCATTGAGATAGTGTGGG*mG *mA*mA |
| 12i-gRNA-3 | RNA | mA*mG*mA*AATCCGTCTTTCATTGACGGGCATTGAGATAGTGTGG*mG *mG*mA |
| 12i-gRNA-4 | RNA | mA*mG*mA*AATCCGTCTTTCATTGACGGCCAGCCTTGCCTTGAC*mC* mA*mA |
| 12i-gRNA-5 | RNA | mA*mG*mA*AATCCGTCTTTCATTGACGGCCTTGTCAAGGCTATTG*mG* mT*mC |
| 12i-gRNA-6 | RNA | mA*mG*mA*AATCCGTCTTTCATTGACGGCCTTGTCAAGGCTATT*mG* mG*mT |
| 12i-gRNA-7 | RNA | mA*mG*mA*AATCCGTCTTTCATTGACGGCAGACAGATATTTGCAT*mT* mG*mA |
| 12i-gRNA-8 | RNA | mA*mG*mA*AATCCGTCTTTCATTGACGGCCCACACTATCTCAATG*mC* mA*mA |
| 12i-gRNA-9 | RNA | mA*mG*mA*AATCCGTCTTTCATTGACGGAGACAGATATTTGCATT*mG* mA*mG |
| 12i-gRNA-10 | RNA | mA*mG*mA*AATCCGTCTTTCATTGACGGTCAAGGCTATTGGTCAA*mG *mG*mC |
| 12i-gRNA-11 | RNA | mA*mG*mA*AATCCGTCTTTCATTGACGGAGCAGCAGTATCCTCTT*mG* mG*mG |
| 12i-gRNA-12 | RNA | mA*mG*mA*AATCCGTCTTTCATTGACGGGTCAAGTTTGCCTTGTC*mA* mA*mG |

In another aspect, the present application provides a nucleic acid molecule encoding the gRNA molecule described in the present application.

In another aspect, the present application provides a vector comprising the nucleic acid molecule described in the present application.

In some embodiments, the vector is selected from lentivirus vectors, adenovirus vectors, adeno-associated virus (AAV) vectors, herpes simplex virus (HSV) vectors, plasmids, minicircles, nanoplasmids and RNA vectors.

In another aspect, the present application provides a ribonucleoprotein (RNP) complex, comprising the gRNA molecule described in the present application and a nuclease of a CRISPR/Cas system.

In some embodiments, the nuclease is a class II type V Cas enzyme.

In some embodiments, the nuclease is a Cas12b nuclease.

In some embodiments, the Cas12b nuclease comprises a variant thereof, an ortholog thereof and/or a functionally active fragment thereof.

In some embodiments, the Cas12b nuclease is a Cas12b nuclease from *Alicyclobacillus acidiphilus* (AaCas12b).

In some embodiments, the AaCas12b nuclease comprises one or more of the following mutations relative to wild-type AaCas12b nuclease: (1) a substitution of one or more amino acid residues in the wild-type AaCas12b nuclease that interact with a protospacer adjacent motif (PAM) with a positively charged amino acid residue(s); (2) a substitution of one or more amino acid residues involved in opening a DNA double strand in the wild-type AaCas12b nuclease with an amino acid residue(s) having an aromatic ring; and/or (3) a substitution of one or more amino acid residues in RuvC domain of the wild-type AaCas12b nuclease that interact with a single-stranded DNA substrate with a positively charged amino acid residue(s) or a hydrophobic amino acid residue(s), wherein the amino acid sequence of the wild-type AaCas12b nuclease is as set forth in SEQ ID NO: 91.

In some embodiments, the one or more amino acid residues that interact with PAM are located at one or more of positions 116, 123, 130, 132, 144, 145, 153, 173, 222, 395, 400 and 475, wherein the amino acid residues are numbered according to SEQ ID NO: 91.

In some embodiments, the substitution of one or more amino acid residues in the wild-type AaCas12b nuclease that interact with PAM with a positively charged amino acid residue(s) is one or more of substitutions D116R and E475R, wherein the amino acid residues are numbered according to SEQ ID NO: 91.

In some embodiments, the one or more amino acid residues involved in opening the DNA double strand are located at one or more of positions 118 and 119, wherein the amino acid residues are numbered according to SEQ ID NO: 91.

In some embodiments, the substitution of one or more amino acid residues involved in opening the DNA double strand in the wild-type AaCas12b nuclease with an amino acid residue(s) having an aromatic ring is substitution Q119Y, Q119F or Q119W, wherein the amino acid residues are numbered according to SEQ ID NO: 91.

In some embodiments, the one or more amino acid residues in RuvC domain that interact with a single-stranded DNA substrate are located at one or more positions 300, 301, 304, 329, 636, 639, 647, 682, 757, 758, 761, 764, 768, 852, 854, 856, 857, 858, 860, 862, 863, 865, 866, 867, 869, 938, 956, 957, 958, 994, 1093 and 1097, wherein the amino acid residues are numbered according to SEQ ID NO: 91.

In some embodiments, the substitution of one or more amino acid residues in RuvC domain of the wild-type AaCas12b nuclease that interact with a single-stranded DNA substrate is one or more of substitutions E636R, Q639R, T647R, Q682R, I757R, E758R, E761R, Q854R, N857K, D858R, I994R, Q1093R and W1097R, wherein the amino acid residues are numbered according to SEQ ID NO: 91.

In some embodiments, the gRNA molecule comprises a nucleotide sequence set forth in any one of SEQ ID NOs: 12-59.

In some embodiments, the nuclease is a Cas12i nuclease.

In some embodiments, the Cas12i nuclease comprises a variant thereof, an ortholog thereof and/or a functionally active fragment thereof.

In some embodiments, the Cas12i nuclease comprises one or more of the following mutations relative to wild-type Cas12i2 nuclease: (1) a substitution of one or more amino acids in the wild-type Cas12i2 nuclease that interact with PAM with a positively charged amino acid(s); (2) a substitution of one or more amino acids involved in opening a DNA double strand in the wild-type Cas12i2 nuclease with an amino acid(s) having an aromatic ring; (3) a substitution of one or more amino acids in RuvC domain of the wild-type Cas12i2 nuclease that interact with a single-stranded DNA substrate with a positively charged amino acid(s); and/or (4) a substitution of one or more amino acids in the wild-type Cas12i2 nuclease that interact with a DNA-RNA duplex with a positively charged amino acid(s), wherein the amino acid sequence of the wild-type Cas12i2 nuclease is as set forth in SEQ ID NO: 92.

In some embodiments, the one or more amino acids that interact with PAM are located at one or more of positions 176, 238, 447 and/or 563, wherein the positions are numbered according to SEQ ID NO: 92.

In some embodiments, the positively charged amino acid in (1) is R or K.

In some embodiments, the Cas12i nuclease comprises any one of the following mutations or combinations of mutations: (1) E563R; (2) E176R, T447R, E176R and E563R; (3) K238R and E563R; (4) E176R, K238R and T447R; (5) E176R, K238R and E563R; (6) E176R, T447R and E563R; and/or (7) E176R, K238R, T447R and E563R, wherein the amino acid residues are numbered according to SEQ ID NO: 92.

In some embodiments, the one or more amino acids involved in opening a DNA double strand are located at one or more of positions 163 and/or 164, wherein the positions are numbered according to SEQ ID NO: 92.

In some embodiments, the amino acid having an aromatic ring in (2) is F, Y or W.

In some embodiments, the substitution of one or more amino acids involved in opening a DNA double strand with an amino acid(s) having an aromatic ring refers to Q163F, Q163Y, Q163W and/or N164F or N164Y, wherein the amino acid residues are numbered according to SEQ ID NO: 92.

In some embodiments, the one or more amino acids in RuvC domain that interact with a single-stranded DNA substrate are located at one or more of positions 323, 362, 425, 925, 926, 391, 424 and/or 929, wherein the positions are numbered according to SEQ ID NO: 92.

In some embodiments, the positively charged amino acid in (3) is R or K.

In some embodiments, the Cas12i nuclease comprises any one of the following mutations or combinations of mutations: (1) E323R; (2) D362R; (3) Q425R; (4) N925R; (5) I926R; (6) E323R and D362R; (7) E323R and Q425R; (8) E323R and I926R; (9) Q425R and I926R; (10) D362R and I926R; (11) N925R and I926R; (12) E323R, D362R and Q425R; (13) E323R, D362R and I926R; (14) E323R, Q425R and I926R; (15) D362R, N925R and I926R; and/or (16) E323R, D362R, Q425R and I926R, wherein the amino acid residues are numbered according to SEQ ID NO: 92.

In some embodiments, the one or more amino acids that interact with a DNA-RNA duplex are located at one or more of positions 116, 117, 159, 161, 319, 343 and/or 958, wherein the positions are numbered according to SEQ ID NO: 92.

In some embodiments, the positively charged amino acid in (4) is R or K.

In some embodiments, the Cas12i nuclease comprises any one of the following mutations or combinations of mutations: G116R, E117R, T159R, S161R, E319R, E343R and/or D958R, wherein the amino acid residues are numbered according to SEQ ID NO: 92.

In some embodiments, the Cas12i nuclease further comprises one or more mutations in a flexible region that are capable of imparting increased flexibility in the flexible region of the Cas12i nuclease as compared to the wild-type Cas12i2 nuclease.

In some embodiments, the flexible region is selected from the group consisting of regions corresponding to amino acid residues 228-232, amino acid residues 439-443, amino acid residues 478-482, amino acid residues 500-504, amino acid residues 775-779, and amino acid residues 925-929, wherein the amino acid residues are numbered according to SEQ ID NO: 92.

In some embodiments, the one or more mutations in a flexible region comprise an insertion of one or more G residues in the flexible region.

In some embodiments, the one or more G residues are inserted N-terminally to a flexible amino acid residue in the flexible region, wherein the flexible amino acid residue is selected from the group consisting of G, S, N, D, H, M, T, E, Q, K, R, A and P.

In some embodiments, the one or more mutations in a flexible region comprises a substitution of a hydrophobic amino acid residue in the flexible region with a G residue, wherein the hydrophobic amino acid residue is selected from the group consisting of L, I, V, C, Y, F and W.

In some embodiments, the one or more mutations in a flexible region are: (1) I926G; and/or (2) 439G or 439GG, wherein the amino acid residues are numbered according to SEQ ID NO: 92.

In some embodiments, the Cas12i nuclease comprises mutations E176R, K238R, T447R, E563R, N164Y, E323R and D362R, wherein the amino acid residues are numbered according to SEQ ID NO: 92.

In some embodiments, the gRNA molecule comprises a nucleotide sequence set forth in any one of SEQ ID NOs: 67-78.

In some embodiments, the N-terminus and/or C-terminus of the nuclease is linked to one or more nuclear localization signals.

In some embodiments, the nuclear localization signal is linked to a His tag.

In some embodiments, the nuclease comprises an amino acid sequence selected from any one of SEQ ID NOs: 1-11.

In another aspect, the present application provides a composition comprising: (1) one or more gRNA molecules according to any one of claims 1-11, and a nuclease of a CRISPR/Cas system; (2) a nucleic acid encoding one or more gRNA molecules according to any one of claims 1-11, and a nuclease of a CRISPR/Cas system; (3) one or more gRNA molecules according to any one of claims 1-11, and a nucleic acid encoding a nuclease of a CRISPR/Cas system; or (4) a nucleic acid encoding one or more gRNA molecules according to any one of claims 1-11, and a nucleic acid encoding a nuclease of a CRISPR/Cas system.

In some embodiments, the one or more gRNA molecules and the nuclease of a CRISPR/Cas system in (1) are present in a ribonucleoprotein (RNP) complex.

In some embodiments, the nuclease is a class II type V Cas enzyme.

In some embodiments, the nuclease is a Cas12b nuclease.

In some embodiments, the Cas12b nuclease comprises a variant thereof, an ortholog thereof and/or a functionally active fragment thereof.

In some embodiments, the Cas12b nuclease is a Cas12b nuclease from *Alicyclobacillus acidiphilus* (AaCas12b).

In some embodiments, the AaCas12b comprises one or more of the following mutations relative to wild-type AaCas12b nuclease: (1) a substitution of one or more amino acid residues in the wild-type AaCas12b nuclease that interact with a protospacer adjacent motif (PAM) with a positively charged amino acid residue(s); (2) a substitution of one or more amino acid residues involved in opening a DNA double strand in the wild-type AaCas12b nuclease with an amino acid residue(s) having an aromatic ring; and/or (3) a substitution of one or more amino acid residues in RuvC domain of the wild-type AaCas12b nuclease that interact with a single-stranded DNA substrate with a positively charged amino acid residue(s) or a hydrophobic amino acid residue(s), wherein the amino acid sequence of the wild-type AaCas12b nuclease is as set forth in SEQ ID NO: 91.

In some embodiments, the one or more amino acid residues that interact with PAM are located at one or more of positions 116, 123, 130, 132, 144, 145, 153, 173, 222, 395, 400 and 475, wherein the amino acid residues are numbered according to SEQ ID NO: 91.

In some embodiments, the substitution of one or more amino acid residues in the wild-type AaCas12b nuclease that interact with PAM with a positively charged amino acid residue(s) is one or more of substitutions D116R and E475R, wherein the amino acid residues are numbered according to SEQ ID NO: 91.

In some embodiments, the one or more amino acid residues involved in opening the DNA double strand are located at one or more of positions 118 and 119, wherein the amino acid residues are numbered according to SEQ ID NO: 87.

In some embodiments, the substitution of one or more amino acid residues involved in opening the DNA double strand in the wild-type AaCas12b nuclease with an amino acid residue(s) having an aromatic ring is substitution Q119Y, Q119F or Q119W, wherein the amino acid residues are numbered according to SEQ ID NO: 91.

In some embodiments, the one or more amino acid residues in RuvC domain that interact with a single-stranded DNA substrate are located at one or more positions 300, 301, 304, 329, 636, 639, 647, 682, 757, 758, 761, 764, 768, 852, 854, 856, 857, 858, 860, 862, 863, 865, 866, 867, 869, 938, 956, 957, 958, 994, 1093 and 1097, wherein the amino acid residues are numbered according to SEQ ID NO: 91.

In some embodiments, the substitution of one or more amino acid residues in RuvC domain of the wild-type AaCas12b nuclease that interact with a single-stranded DNA substrate is one or more of substitutions E636R, Q639R, T647R, Q682R, I757R, E758R, E761R, Q854R, N857K, D858R, I994R, Q1093R and W1097R, wherein the amino acid residues are numbered according to SEQ ID NO: 91.

In some embodiments, the gRNA molecule comprises a nucleotide sequence set forth in any one of SEQ ID NOs: 12-59.

In some embodiments, the nuclease is a Cas12i nuclease.

In some embodiments, the Cas12i nuclease comprises a variant thereof, an ortholog thereof and/or a functionally active fragment thereof.

In some embodiments, the Cas12i nuclease comprises one or more of the following mutations relative to wild-type Cas12i2 nuclease: (1) a substitution of one or more amino acids in the wild-type Cas12i2 nuclease that interact with PAM with a positively charged amino acid(s); (2) a substitution of one or more amino acids involved in opening a DNA double strand in the wild-type Cas12i2 nuclease with an amino acid(s) having an aromatic ring; (3) a substitution of one or more amino acids in RuvC domain of the wild-type Cas12i2 nuclease that interact with a single-stranded DNA substrate with a positively charged amino acid(s); and/or (4) a substitution of one or more amino acids in the wild-type Cas12i2 nuclease that interact with a DNA-RNA duplex with a positively charged amino acid(s), wherein the amino acid sequence of the wild-type Cas12i2 nuclease is as set forth in SEQ ID NO: 92.

In some embodiments, the one or more amino acids that interact with PAM are located at one or more of positions 176, 238, 447 and/or 563, wherein the positions are numbered according to SEQ ID NO: 92.

In some embodiments, the positively charged amino acid in (1) is R or K.

In some embodiments, the Cas12i nuclease comprises any one of the following mutations or combinations of mutations: (1) E563R; (2) E176R, T447R, E176R and E563R; (3) K238R and E563R; (4) E176R, K238R and T447R; (5) E176R, K238R and E563R; (6) E176R, T447R and E563R; and/or (7) E176R, K238R, T447R and E563R, wherein the amino acid residues are numbered according to SEQ ID NO: 92.

In some embodiments, the one or more amino acids involved in opening a DNA double strand are located at one or more of positions 163 and/or 164, wherein the positions are numbered according to SEQ ID NO: 92.

In some embodiments, the amino acid having an aromatic ring in (2) is F, Y or W.

In some embodiments, the substitution of one or more amino acids involved in opening a DNA double strand with an amino acid(s) having an aromatic ring refers to Q163F, Q163Y, Q163W and/or N164F or N164Y, wherein the amino acid residues are numbered according to SEQ ID NO: 92.

In some embodiments, the one or more amino acids in RuvC domain that interact with a single-stranded DNA substrate are located at one or more of positions 323, 362, 425, 925, 926, 391, 424 and/or 929, wherein the positions are numbered according to SEQ ID NO: 92.

In some embodiments, the positively charged amino acid in (3) is R or K.

In some embodiments, the Cas12i nuclease comprises any one of the following mutations or combinations of mutations: (1) E323R; (2) D362R; (3) Q425R; (4) N925R; (5) I926R; (6) E323R and D362R; (7) E323R and Q425R; (8) E323R and I926R; (9) Q425R and I926R; (10) D362R and I926R; (11) N925R and I926R; (12) E323R, D362R and Q425R; (13) E323R, D362R and I926R; (14) E323R, Q425R and I926R; (15) D362R, N925R and I926R; and/or (16) E323R, D362R, Q425R and I926R, wherein the amino acid residues are numbered according to SEQ ID NO: 92.

In some embodiments, the one or more amino acids that interact with a DNA-RNA duplex are located at one or more of positions 116, 117, 159, 161, 319, 343 and/or 958, wherein the positions are numbered according to SEQ ID NO: 92.

In some embodiments, the positively charged amino acid in (4) is R or K.

In some embodiments, the Cas12i nuclease comprises any one of the following mutations or combinations of mutations: G116R, E117R, T159R, S161R, E319R, E343R and/or D958R, wherein the amino acid residues are numbered according to SEQ ID NO: 92.

In some embodiments, the Cas12i nuclease further comprises one or more mutations in a flexible region that are capable of imparting increased flexibility in the flexible region of the Cas12i nuclease as compared to the wild-type Cas12i2 nuclease.

In some embodiments, the flexible region is selected from the group consisting of regions corresponding to amino acid residues 228-232, amino acid residues 439-443, amino acid residues 478-482, amino acid residues 500-504, amino acid residues 775-779, and amino acid residues 925-929, wherein the amino acid residues are numbered according to SEQ ID NO: 92.

In some embodiments, the one or more mutations in a flexible region comprise an insertion of one or more G residues in the flexible region.

In some embodiments, the one or more G residues are inserted N-terminally to a flexible amino acid residue in the flexible region, wherein the flexible amino acid residue is selected from the group consisting of G, S, N, D, H, M, T, E, Q, K, R, A and P.

In some embodiments, the one or more mutations in a flexible region comprises a substitution of a hydrophobic amino acid residue in the flexible region with a G residue, wherein the hydrophobic amino acid residue is selected from the group consisting of L, I, V, C, Y, F and W.

In some embodiments, the one or more mutations in a flexible region are: (1) I926G; and/or (2) 439G or 439GG, wherein the amino acid residues are numbered according to SEQ ID NO: 92.

In some embodiments, the Cas12i nuclease comprises mutations E176R, K238R, T447R, E563R, N164Y, E323R and D362R, wherein the amino acid residues are numbered according to SEQ ID NO: 92.

In some embodiments, the gRNA molecule comprises a nucleotide sequence set forth in any one of SEQ ID NOs: 67-78.

In some embodiments, the N-terminus and/or C-terminus of the nuclease is linked to one or more nuclear localization signals.

In some embodiments, the nuclear localization signal is linked to a His tag.

In some embodiments, the nuclease comprises an amino acid sequence selected from any one of SEQ ID NOs: 1-11.

In some embodiments, the composition further optionally comprises a pharmaceutically acceptable carrier.

In another aspect, the present application provides a cell comprising the gRNA molecule described in the present application, the nucleic acid molecule described in the present application, the vector described in the present application, the ribonucleoprotein (RNP) complex described in the present application and/or the composition described in the present application.

In another aspect, the present application provides a cell population or progeny thereof, comprising a gene product produced by administration of the ribonucleoprotein (RNP) complex described in the present application, the composition described in the present application, or the cell described in the present application.

In some embodiments, the gene product comprises an HBG gene having a modification.

In some embodiments, the modification is a base insertion or deletion (indel) at or near a target sequence, wherein the target sequence is located within an HBG1 promoter region between chr11: 5,248,269 and 5,249,857 of human genome hg19, or within an HBG2 promoter region between chr11: 5,253,188 and 5,254,781 of human genome hg19.

In some embodiments, the target sequence is located between about 210 bp upstream and about 100 bp upstream of transcription initiation sites of the HBG1 and HBG2.

In some embodiments, the cell population is capable of differentiating into differentiated cells of an erythroid cell lineage, and the differentiated cells have an increased level of fetal hemoglobin expression as compared to a cell population in which the HBG gene is not modified.

In another aspect, the present application provides a kit, comprising the gRNA molecule described in the present application, the nucleic acid molecule described in the present application, the vector described in the present application, the ribonucleoprotein (RNP) complex described in the present application, the composition described in the present application, the cell described in the present application, and/or the cell population or progeny thereof described in the present application.

In another aspect, the present application provides a method for regulating fetal hemoglobin (HbF) expression in a cell, cell population or progeny thereof, comprising administering the ribonucleoprotein (RNP) complex described in the present application, the composition described in the present application, the cell described in the present application, the cell population or progeny thereof described in the present application, and/or the kit described in the present application.

In some embodiments, the cell or cell population is a hematopoietic stem/progenitor cell (HSPC).

In some embodiments, the cell or cell population is CD34+ HSPC.

In another aspect, the present application provides a method for treating, preventing or alleviating a hemoglobinopathy and/or related condition thereof, comprising administering to a subject in need thereof an effective amount of the ribonucleoprotein (RNP) complex described in the present application, the composition described in the present application, the cell described in the present application, the cell population or progeny thereof described in the present application, and/or the kit described in the present application.

In some embodiments, the hemoglobinopathy and/or related condition thereof is selected from: sickle cell disease, sickle cell anemia, hemoglobin C disease, hemoglobin C trait, hemoglobin S/C disease, hemoglobin D disease, hemoglobin E disease, thalassemia, a hemoglobinopathy with decreased oxygen affinity, and an unstable hemoglobinopathy.

In another aspect, the present application provides use of the gRNA molecule described in the present application, the nucleic acid molecule described in the present application, the vector described in the present application, the ribonucleoprotein (RNP) complex described in the present application, the composition described in the present application, the cell described in the present application, and/or the cell population or progeny thereof described in the present application in the manufacture of a medicament for the treatment of a hemoglobinopathy and/or related condition thereof.

In another aspect, the present application provides the gRNA molecule described in the present application, the nucleic acid molecule described in the present application, the vector described in the present application, the ribonucleoprotein (RNP) complex described in the present application, the composition described in the present application, the cell described in the present application, the cell population or progeny thereof described in the present application, and/or the kit described in the present application, for use in the treatment of a hemoglobinopathy and/or related condition thereof.

Those skilled in the art can easily discern other aspects and advantages of the present application from the detailed description below. Only exemplary embodiments of the present application are shown and described in the following detailed description. As will be appreciated by those skilled in the art, the contents of the present application enable those skilled in the art to make changes to the specific embodiments disclosed without departing from the spirit and scope of the invention to which the present application relates. Accordingly, the description in the drawings and specification of the present application is merely exemplary and not limiting.

### Brief Description of the Drawings

The specific features of the invention to which the present application relates are as shown in the appended claims. The features and advantages of the invention to which the present application relates can be better understood by reference to the exemplary embodiments and drawings described in detail below. A brief description of the drawings is as follows:
FIG. 1A shows the distribution of key motifs and the gRNA-targeted locations within the HBG promoter regions. FIGs. 1B-1C show the screening results of gRNAs with a binding region length of 20 nt; and FIGs. 1D-1E show the screening results of crRNAs and sgRNAs with different binding region lengths.
FIG. 2A shows structural diagrams of eight Cas12b^{Max} constructs. FIGs. 2B-2C show the editing efficiency and HbF+ erythroid cell induction efficiency of Cas12b^{Max} constructs M1-M7 guided by sgRNA1. FIG. 2D shows the cleavage activity of Cas12b^{Max} constructs M1 and M2 compared to SpCas9 for the HBG promoter region. FIG. 2E shows the editing efficiency of construct M1 and construct M8 from different suppliers for hematopoietic stem cell target genes under different RNP dose conditions.
FIGs. 3A-3C show the optimization scheme and screening results of the sgRNA single-molecule scaffold for guiding Cas12b^{Max}.
FIGs. 4A-4D show the screening results of RNP doses and Cas enzyme : sgRNA ratios, the editing efficiency in hematopoietic stem cells of healthy controls and thalassemia patients, the main edited sequences in the target genomic region, and the statistical distribution results of lengths of edited sequences, respectively, when the Cas12b^{Max}-sgRNA G1 complex (RNP) was used to edit hematopoietic stem cells.
FIGs. 5A-5B show evaluation of off-target effects of the Cas12b^{Max}-sgRNA G1 complex (RNP) in editing hematopoietic stem cells.
FIGs. 6A-6D show the induction results of the Cas12b^{Max}-sgRNA G1 complex (RNP) on mRNA levels of γ-globin and fetal hemoglobin, as well as HbF-positive erythroid cell levels and protein levels.
FIGs. 7A-7B show the effects of the Cas12b^{Max}-sgRNA G1 complex (RNP) on differentiation potential and cell innate immunity of hematopoietic stem cells during in vitro editing.
FIGs. 8A-8F show the in vivo efficacy evaluation of hematopoietic stem cells edited by the Cas12b^{Max}-sgRNA G1 complex (RNP).

### Detailed Description of Embodiments

The implementation of the invention of the present application is illustrated by the specific embodiments below. Those familiar with this technology can easily understand other advantages and effects of the invention of the present application from the contents disclosed in the specification.

### Definitions of Terms

In the present application, the term "guide RNA" may be used interchangeably with "guide RNA (molecule)" and "gRNA (molecule)", and generally refers to a group of nucleic acid molecules that facilitate the specific guidance of an RNA-guided nuclease or other effector molecule (usually in complex with a gRNA molecule) onto a target sequence. In some embodiments, such guidance is achieved by hybridizing a portion of the gRNA to DNA (e.g., via a gRNA targeting domain) and by binding a portion of the gRNA molecule with an RNA-guided nuclease or other effector molecule (e.g., via at least a tracrRNA). In certain embodiments, a gRNA molecule consisting of a single contiguous polynucleotide molecule, is referred to in the present application as a "single guide RNA" or "sgRNA", or the like. In other embodiments, a gRNA molecule consisting of a plurality, typically two, polynucleotide molecules that are capable of associating with each other, typically by hybridization, is referred to herein as "dual guide RNA" or "dgRNA" or the like. A gRNA molecule typically comprises a targeting domain and a tracr. In an embodiment, the targeting domain and tracr are arranged on a single polynucleotide. In other embodiments, the targeting domain and the tracr are arranged on separate polynucleotides.

In the present application, the term "target sequence" generally refers to a nucleic acid sequence that is complementary, e.g. fully complementary, to a gRNA targeting domain. In an embodiment, a target sequence is arranged on a genomic DNA. In one embodiment, the target sequence is adjacent to a protospacer adjacent motif (PAM) sequence recognized by a protein having nuclease or other effector activity (such as a PAM sequence recognized by a Cas enzyme), on the same strand of DNA or on the complementary strand of DNA. In an embodiment, the target sequence is a target sequence within a gene or locus that affects globin gene expression, for example, β-globin or fetal hemoglobin (HbF) expression. In an embodiment, the target sequence is a target sequence within a non-deletional HPFH region, for example within the HBG1 and/or HBG2 promoter region. In contrast, in the present application, the term "targeting domain" (when the term is used in conjunction with a gRNA) refers to the portion of a gRNA molecule that recognizes a target sequence (e.g., a target sequence within a nucleic acid, e.g., within a gene, of a cell), e.g., is complementary to the target sequence.

In the present application, the term "crRNA" (when the term is used in conjunction with a gRNA molecule) generally refers to the portion of a gRNA molecule that comprises a targeting domain and interacts with a tracr to form a dgRNA domain.

In the present application, the term "tracr" (when the term is used in conjunction with a gRNA molecule) generally refers to the portion of a gRNA that binds to a nuclease or other effector molecule. In an embodiment, the tracr comprises a nucleic acid sequence that specifically binds to a Cas enzyme. In an embodiment, the tracr comprises a nucleic acid sequence forming a portion of a dgRNA.

The term "CRISPR system", "Cas system" or "CRISPR/Cas system" refers to a group of molecules comprising an RNA-guided nuclease or other effector molecule and a gRNA molecule, which together are necessary and sufficient to guide and effect modification of a nucleic acid by an RNA-guided nuclease or other effector molecule at a target sequence. In one embodiment, the CRISPR system comprises a gRNA and a Cas protein (e.g., a Cas9 protein), and such a system comprising Cas9 or a modified Cas9 molecule is referred to as a "Cas9 system" or "CRISPR/Cas9 system". In one example, the gRNA molecule and the Cas molecule may be complexed to form a ribonucleoprotein (RNP) complex.

In the present application, the term "indel" generally refers to a nucleic acid comprising one or more nucleotide insertions, one or more nucleotide deletions, or a combination of nucleotide insertions and deletions relative to a reference nucleic acid, that is generated after exposure to a composition comprising a gRNA molecule (e.g., a CRISPR system). An insertion/deletion can be determined by sequencing nucleic acids, for example by NGS, after exposure to a composition comprising a gRNA molecule. With respect to an insertion/deletion site, an insertion/deletion is said to be "at or near" a reference site (e.g., a site complementary to a targeting domain of a gRNA molecule) if the insertion/deletion comprises at least one insertion or deletion within about 10, about 20, about 30, about 40, about 50, about 60, about 70, about 80, about 90, or about 100 nucleotides of the reference site, or the insertion/deletion overlaps partially or fully with the reference site (e.g., comprises at least one insertion or deletion that overlaps with about 10, about 20, about 30, about 40, about 50, about 60, about 70, about 80, about 90, or about 100 nucleotides of a site complementary to the targeting domain of a gRNA molecule described herein, or at least one insertion or deletion within about 10, about 20, about 30, about 40, about 50, about 60, about 70, about 80, about 90, or about 100 nucleotides of a site complementary to the targeting domain of a gRNA molecule described herein). For example, the indel described in the present application may be an insertion of 10, 11, 12, 13, 14, 15, 16, 17, 18, 19 or 20 nucleotides at or near a target sequence; or may be a deletion of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides at or near a target sequence.

In the present application, the term "functionally active fragment" generally refers to a fragment that has a partial region of a full-length protein or nucleic acid, but retains or partially retains the biological activity or function of the full-length protein or nucleic acid. For example, a functionally active fragment may retain or partially retain the ability of a full-length protein to bind to another molecule.

In the present application, the term "nuclear localization sequence" or "nuclear localization signal" or "NLS" generally refers to a peptide that directs a protein to the nucleus of a cell. For example, the NLS can be located anywhere on a peptide chain. NLS may differ in length and/or sequence, and many specific NLS sequences have been described. For example, it has now been found that peptides comprising positively charged amino acids, especially lysine (K), arginine (R) and/or histidine (H), can generally be used as NLS. Thus, an exemplary NLS may be, for example, a peptide of 4 to 20 amino acids, more specifically 4 to 15, 4 to 12, 4 to 10 or 4 to 8 amino acids, in which at least 4 amino acids (more specifically 60%, 70%, 75%, 80%, 85% or 90% of the amino acid residues in the NLS peptide) are positively charged amino acids preferably selected from K, R or H. NLS sequences that can be used with the nucleases and/or constructs thereof described in the present application are known in the art. Non-limiting examples of such NLS sequences include nucleoplasmin NLS having the amino acid sequence set forth below (KRPAATKKAGQAKKKK), simian virus 40 ("SV40") NLS (PKKKRKV), c-Myc NLS, and/or BP SV40 NLS.

In the present application, the term "His-tag" generally refers to an epitope tag consisting of 6 to 10 histidine residues that is readily recognized by tag-specific antibodies. His-tag is one of the commonly used tags for protein purification and detection. Because of its small molecular weight (only 0.84 KD), fusion to a recombinant protein will not affect the structure and biochemical characteristics of the tagged protein. Anti-His antibodies can be used for detection of expression and intracellular localization of His-tagged fusion proteins, for the purification, qualitative, and quantitative detection of His-tagged fusion proteins, and the like, and have a very wide range of applications.

In the present application, the term "nucleic acid" is used interchangeably with "polynucleotide", "nucleotide", "nucleotide sequence" and "oligonucleotide", and generally refers to a nucleotide (e.g., a deoxyribonucleotide or ribonucleotide) and a polymer thereof in a single-, double-, or multi-stranded form or a complement thereof. For example, the nucleotide may be a ribonucleotide, a deoxyribonucleotide, or a modified version thereof. For example, the nucleotide can be single-stranded and double-stranded DNA, single-stranded and double-stranded RNA, and a hybrid molecule comprising a mixture of single-stranded and double-stranded DNA and RNA. For example, the nucleotide can include, but are not limited to, any type of RNA, such as mRNA, siRNA, miRNA, sgRNA, and guide RNA, as well as any type of DNA, genomic DNA, plasmid DNA, and minicircle DNA, and any fragment thereof. The term also encompasses nucleic acids containing known nucleotide analogs or modified backbone residues or bonds, and the nucleic acids are synthetic, naturally occurring, and non-naturally occurring.

In the present application, the term "sequence encoding ..." or "nucleic acid encoding ..." generally refers to a nucleic acid (an RNA or DNA molecule) comprising a nucleotide sequence encoding a protein. The coding sequence may also comprise start and stop signals operably linked to regulatory elements comprising a promoter and a polyadenylation signal capable of directing expression in cells of an individual or mammal to which the nucleic acid has been administered. The coding sequence can be codon optimized.

In the present application, the term "pharmaceutically acceptable carrier" generally refers to a carrier for administration of a therapeutic agent, such as an antibody or polypeptide, a gene, and other therapeutic agents. The term refers to any pharmaceutical carrier that does not itself induce the production of antibodies harmful to the individual receiving the composition and that can be administered without undue toxicity. Suitable carriers can be large, slowly metabolized macromolecules, such as proteins, polysaccharides, polylactic acid, polyglycolic acid, polyamino acids, amino acid copolymers, lipid aggregates, and inactivated virus particles. These carriers are well known to those skilled in the art. Pharmaceutically acceptable carriers in therapeutic compositions may include liquids such as water, saline, glycerol, and ethanol. Auxiliary substances such as wetting agents or emulsifiers, and pH buffering substances may also be present in these carriers.

In the present application, the term "kit" generally refers to any collection of two or more components that together constitute a functional unit that can be used for a particular purpose. By way of illustration (without limitation), a kit according to the present disclosure may include a guide RNA complexed with or capable of complexing with an RNA-guided nuclease, together with a pharmaceutically acceptable carrier (e.g., which is suspended in or can be suspended in the carrier). The kit can be used to introduce a complex into, for example, a cell or a subject for the purpose of causing a desired genomic change in such cell or subject. The components of the kit may be packaged together, or the components may be packaged separately. The kit according to the present disclosure optionally further comprises instructions for use describing, for example, the use of the kit according to the method of the present application. The instructions for use may be physically packaged with the kit or may be made available to a user of the kit, for example by electronic means.

In the present application, the term "cell population" generally refers to eukaryotic mammalian (preferably human) cells isolated from a biological source (e.g., blood products or tissues) and derived from more than one type of cell.

In the present application, the terms "hematopoietic stem cells and progenitor cells" or "HSPC" are used interchangeably and refer to a cell population comprising both hematopoietic stem cells ("HSC") and hematopoietic progenitor cells ("HPC"). Such cells are characterized as, for example, CD34+. In an exemplary embodiment, the HSPCs are isolated from bone marrow. In other exemplary embodiments, the HSPCs are isolated from peripheral blood. In other exemplary embodiments, the HSPCs are isolated from cord blood. In one embodiment, the HSPCs are characterized as CD34+/CD38-/CD90+/CD45RA-. In an embodiment, the HSPCs are characterized as CD34+/CD90+/CD49f+ cells. In an embodiment, the HSPCs are characterized as CD34+ cells. In an embodiment, the HSPCs are characterized as CD34+/CD90+ cells. In an embodiment, the HSPCs are characterized as CD34+/CD90+/CD45RA- cells.

In the present application, the term "treating", e.g., a disease, means that a subject (e.g., a human) suffering from, at risk of developing, and/or experiencing symptoms of a disease will experience milder symptoms and/or recover faster in one embodiment upon administration of, for example, the complex, composition, cell, or the like described herein, compared to when the complex, composition, cell, or the like is never administered. In some embodiments, "treating" may refer to reducing or alleviating the progression, severity, and/or duration of a condition, such as a hemoglobinopathy, or alleviating one or more symptoms (preferably, one or more discernible symptoms) of a condition, such as a hemoglobinopathy, resulting from administration of one or more therapies (e.g., one or more therapeutic agents, such as the gRNA molecules, CRISPR systems, or modified cells of the present invention). In particular embodiments, the term "treating" refers to ameliorating at least one measurable physical parameter of a hemoglobinopathy condition, which is not discernible by the patient. In other embodiments, the term "treating" refers to inhibiting progression of a condition physically by, for example, stabilizing discernible symptoms, physiologically by, for example, stabilizing physical parameters, or both. In other embodiments, the term "treating" refers to reducing or stabilizing symptoms of hemoglobinopathies such as sickle cell disease or β-thalassemia.

In the present application, the term "subject" generally refers to animals, typically mammals, such as humans, non-human primates (apes, gibbons, gorillas, chimpanzees, orangutans, macaques), domestic animals (dogs and cats), farm animals (poultry such as chickens and ducks, horses, cows, goats, sheep, pigs), and experimental animals (mice, rats, rabbits, guinea pigs). Human subjects include fetal, neonatal, infant, adolescent, and adult subjects. Subjects include animal disease models, such as mice and other animal models of blood clotting diseases such as HeA, and other animal models known to those skilled in the art.

In the present application, the term "comprise" generally refers to the inclusion of explicitly specified features, but not excluding other elements.

In the present application, the term "selected from" generally refers to encompassing the selected objects and all combinations thereof. For example, "selected from(:) A, B and C" means including all combinations of A, B and C, for example, A, B, C, A + B, A + C, B + C, or A + B + C.

Without wishing to be bound by any theory, the examples below are only intended to illustrate the fusion protein, preparation method and use, etc. of the present application, and are not intended to limit the scope of the invention of the present application.

### Examples

### Experimental materials and methods:

### (1) Cell culture

Human CD34+ HSPCs were obtained from mobilized peripheral blood of identified healthy donors, and CD34+ HSPCs from patients with β-thalassemia were isolated from plerixafor-mobilized peripheral blood. CD34+ HSPCs were enriched using the Miltenyi CD34 Microbead Kit (Miltenyi Biotec). CD34+ HSPCs were maintained and expanded in StemSpan^{™} SFEM II (Stemcell Technologies). The electroporated HSPCs were subjected to erythroid differentiation in vitro following a three-step, serum-free suspension culture protocol. Erythroid phenotype and γ-globin induction were evaluated on day 18.

Semisolid cell culture for colony-forming unit assay: CD34+ HSPCs were seeded into MethoCult^{™} medium (Stemcell Technologies) and cultured in culture dishes. On day 14 of semisolid culture, HSPC-derived colonies were counted or picked for further evaluation.

### (2) RNP electroporation

Chemically synthesized sgRNAs (first 3 nucleotides and last 3 nucleotides modified with 2-O-methyl and 3' phosphorothioate) were purchased from IDT Technologies. AaCas12b variants were expressed and purified by Celllnuo Biotec corporation (prokaryotic expression sequences set forth in SEQ ID NOs: 79-86). When performing electroporation with 20 µl Nucleocuvette Strips, Cas12b (60 to 180 pmol) and sgRNA or dgRNA were mixed in a specific ratio and incubated at room temperature for 15 min, then RNP complexes were prepared. 5 × 10⁴ to 5 × 10⁵ CD34+ HSPCs were resuspended in 20 µl of P3 solution and mixed with RNPs. 20 µl of the HSPCs/RNP mixture was transferred to a cuvette and electroporated using the DZ-100 program. The electroporated cells were transferred to SFEM II medium and cultured overnight, followed by in vitro differentiation or transplantation experiments.

### (3) Measurement of indel frequency using next-generation sequencing technology (NGS)

HSPC genomic DNA was extracted using a mouse tail DNA extraction kit (Beyotime, D7283M). The promoter regions upstream of the transcription initiation sites of HBG1 and HBG2 were amplified in a two-step method using Phanta Max Super-Fidelity DNA polymerase (Vazyme, P525-03) and primers. The PCR products were subjected to NGS sequencing. Sequencing data were imported into CRISPR RGEN Tools Cas-Analyzer online software for indel frequency measurement and indel pattern identification with a 70-bp resolution window.

### (4) RT-qPCR quantification

Total RNA was isolated from HSPCs using the FastPure Cell/Tissue Total RNA Isolation Kit V2 (Vazyme, RC112-01), followed by reverse transcription using the HiScript II 1st Strand cDNA Synthesis Kit (Vazyme, R212-01). RT-qPCR was performed using the AceQ Universal SYBR qPCR Aster Mix (Vazyme, Q511-02).

### (5) Measurement of HbF+ cells by a flow cytometer

Following staining with a human CD235a+ antibody (clone GA-R2 with PE; BD Pharmingen), the in vitro differentiated cells were fixed with 4% paraformaldehyde for 10 min and then permeabilized with 0.1% Triton X-100 at room temperature for 5 min. Subsequently, the cells were stained with a human HbF antibody (clone HBF-1 conjugated to APC; purchased from Invitrogen) in the dark for 30 min. Cells were washed twice to remove unbound antibodies prior to FACS analysis.

### (6) Determination of hemoglobin by HPLC

Hemolysates were prepared using a lysis reagent from erythroid cells differentiated in vitro from CD34+ HSPCs. The lysates were analyzed using a D-10 hemoglobin analyzer (Bio-Rad) and clinically calibrated human hemoglobin was used as a standard.

### Example 1

### Screening of Cas12b/Cas12i constructs and their gRNA-targeted binding regions

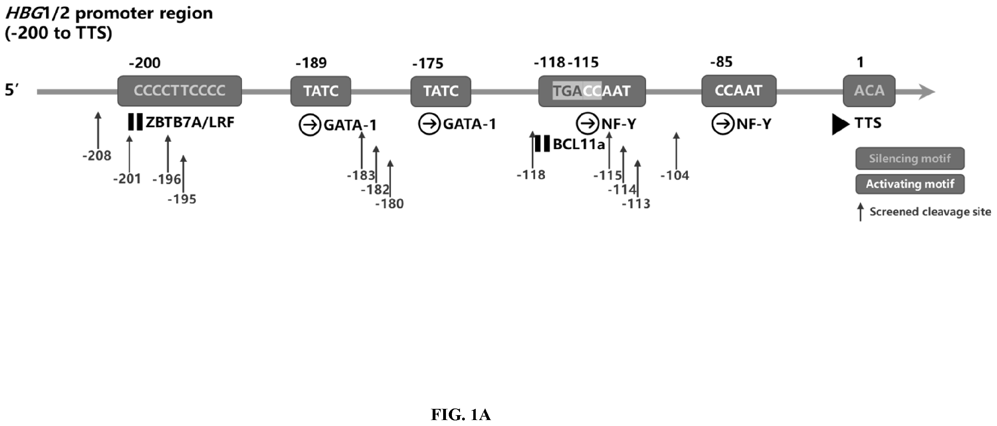

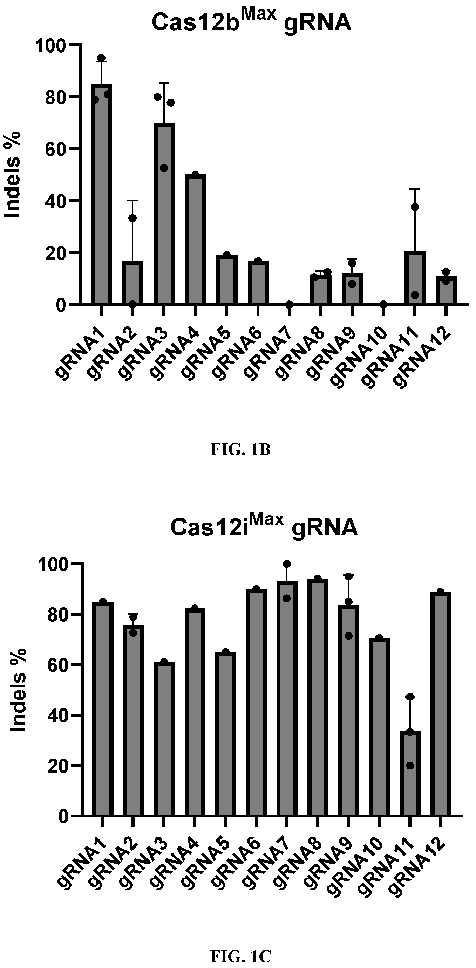

Expression plasmids of Cas12b^{Max} or Cas12i2^{Max} were co-transfected with gRNA expression plasmids into HEK293 cells to screen for gRNAs that can effectively target the cis-elements upstream of the TSS of HBG1 and HBG2, which are then used to switch the expression of β/γ-globin in mature erythrocytes. FIG. 1A shows the genomic locations of the target sites for CRISPR-Cas12 DNA endonucleases to generate HPFH-like mutations in the promoter regions of HBG1 and HBG2 (the human γ-globin locus), with the expected cleavage sites indicated by arrows. The screening results of sgRNA sequences for Cas12b^{Max} are shown in SEQ ID NOs: 12-59. FIG. 1B shows the highly efficient editing gRNAs that target the HBG1/2 original gene region, screened from Cas12b^{Max} gRNAs in the 293HEK cell line. The bimolecular gRNA of Cas12b^{Max} consists of a tracrRNA and a crRNA, and the sequence screening results are shown in SEQ ID NOs: 60-66. FIG. 1C shows the screening results of highly efficient editing by Cas12i^{Max} gRNAs that target the HBG1/2 original region in the 293HEK cell line. The screening results of sgRNA sequences for Cas12i2^{Max} are shown in SEQ ID NOs: 67-78.

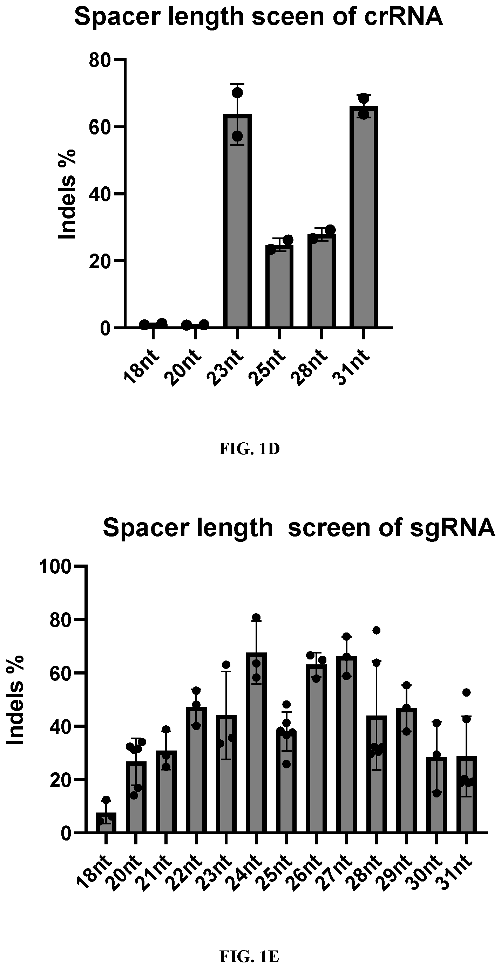

In addition, to improve the editing efficiency of the target genome responsible for erythroid γ-globin expression, single-molecule gRNAs or dual-molecule gRNAs with different spacer lengths generated by in vitro transcription were used to optimize RNP-mediated disruption of the LRF binding motif in the HBG1/2 promoter region in primary human CD34+ HSPCs. NGS data revealed that the targeted indel frequency in HSPCs electroporated with RNPs was correlated with the spacer length of the sgRNA (FIGs. 1D and 1E).

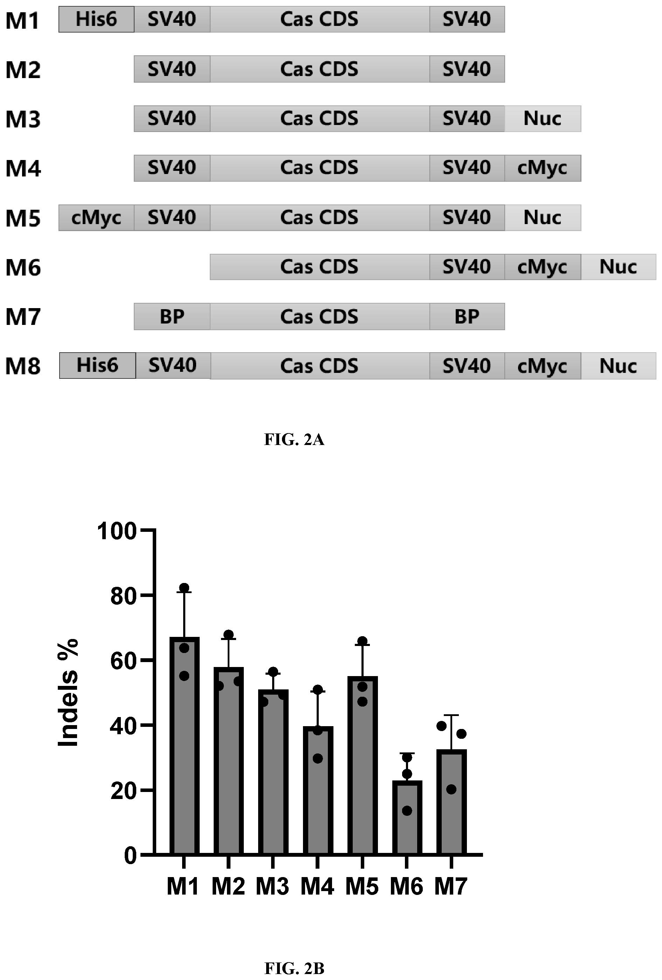

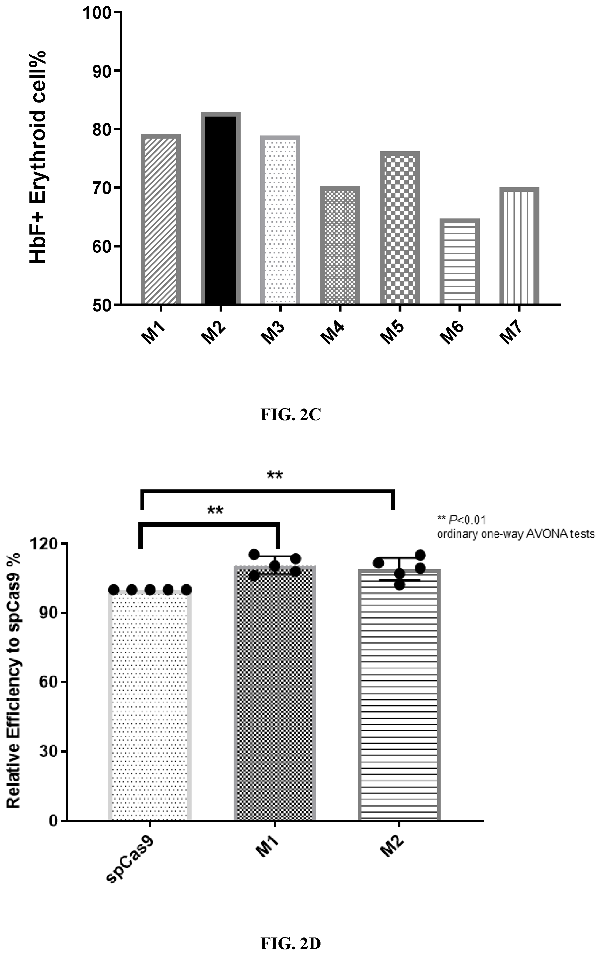

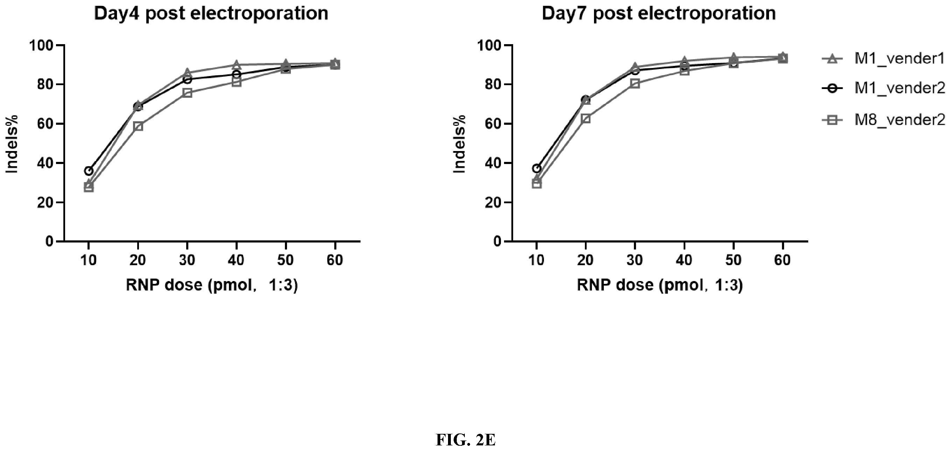

Further optimization introduced a set of engineered Cas protein constructs with different arrangements and combinations of NLS at their ends. The amino acid sequences of these constructs were identified as SEQ ID NOs: 1-7. The experimental results showed that electroporation of RNPs containing different NLS arrangements and combinations at a dose of 3 µM into HSPCs resulted in different editing efficiencies at the target genome, ranging from 30% to 85% (FIG. 2B). Similar magnitudes of HbF induction were detected by FACS among erythroid cells differentiated in vitro from electroporated HSPCs (FIG. 2C). This example also selected the M1 and M2 constructs with high editing efficiency at the target genome for comparison with SpCas9. The results showed that the RNPs provided in the present application exhibited superior editing efficiency to Cas9 (FIG. 2D). Based on the above results at both the protein and RNA levels, combined with comparison of the recovery rates among Cas protein purification schemes, we further introduced the optimized Cas protein construct M8, of which the amino acid sequence was identified as SEQ ID NO: 8. In this example, the M1 construct and the new M8 construct were chosen and compared. The results showed that RNPs of M1 and M8 exhibited similar target gene editing efficiencies in the tested hematopoietic stem cells under different RNP electroporation dosage conditions. (FIG. 2E)

### Example 2

### Optimizing and screening results of the single-molecule sgRNA scaffold for Cas12b^{Max}

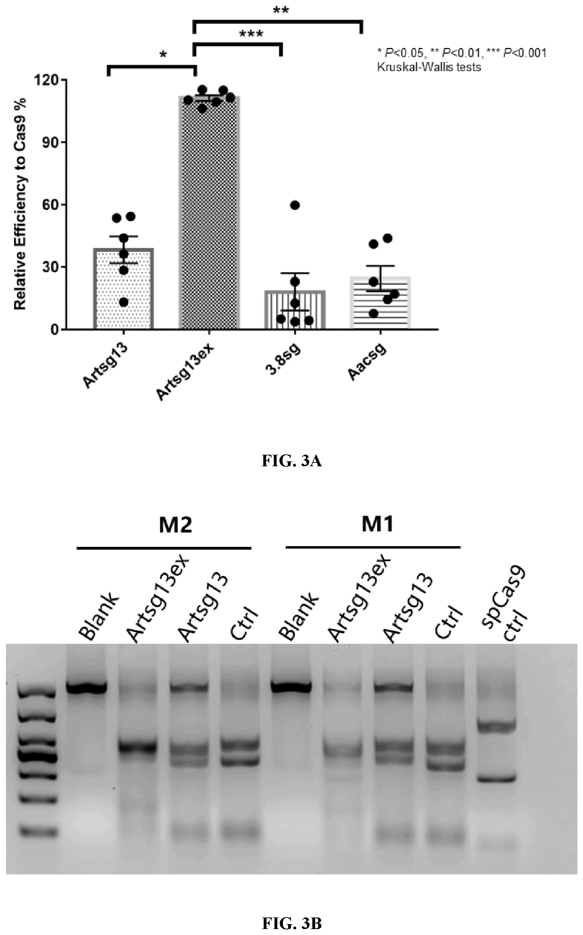

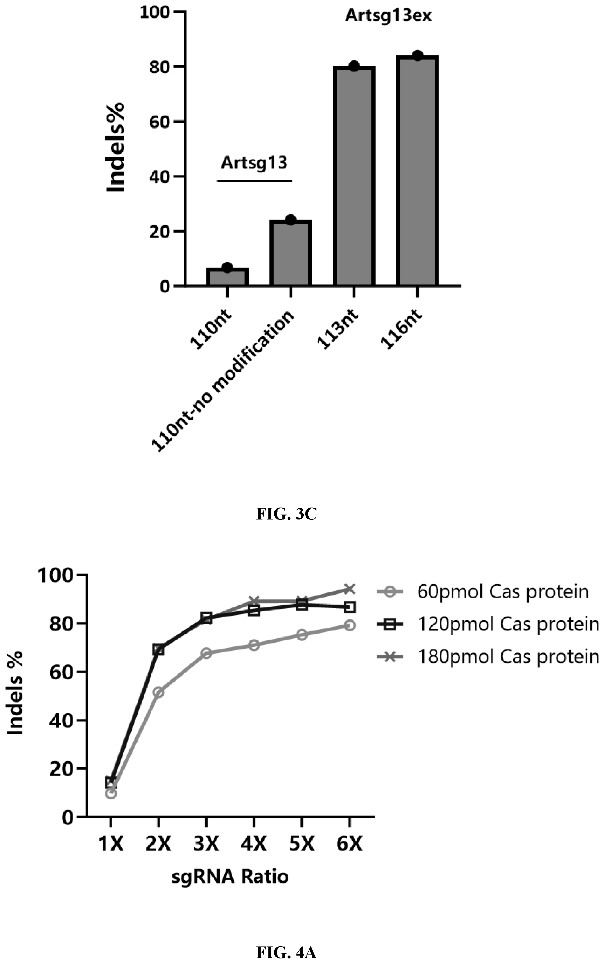

In this example, the in vitro cleavage activities of Cas12b^{Max} complexes comprising Cas12b^{Max} and different sgRNA scaffolds targeting the HBG promoter region were tested (FIG. 3A), as well as the different effects of end-extension schemes of different Artsg13 scaffold sequences on the in vitro cleavage activity of Cas12b^{Max} complexes targeting the HBG promoter region (FIG. 3B). The preferred end-extension scheme for the Artsg13 scaffold sequence was finally obtained: 113 nt dual-end extension or 116 nt single-end extension. The editing efficiency compared with the unoptimized scheme is shown in FIG. 3C. The 110 nt-nm sequence and the 113 nt sequence of FIG. 3C are as set forth in SEQ ID NOs: 13 and 90 (the sequence set forth in SEQ ID NO: 90 is chemically modified:

mG*mG*mT*CGTCTATAGGACGGCGAGTTTTTCAACGGGTGTGCCAATGGCCAC

TTTCCAGGTGGCAAAGCCCGTTGAGCTTCAAAGAAGTGGCACAGATAGTGTGGGGA
AGGGGC*mC*mC*mC).

### Example 3

### Gene editing of hematopoietic stem cells by the selected Cas12b^{Max} and sgRNA-G1

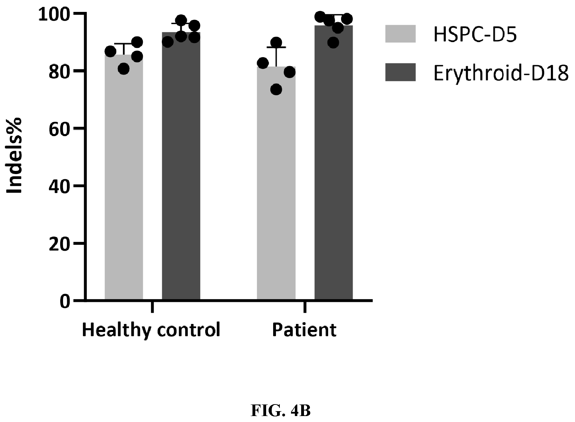

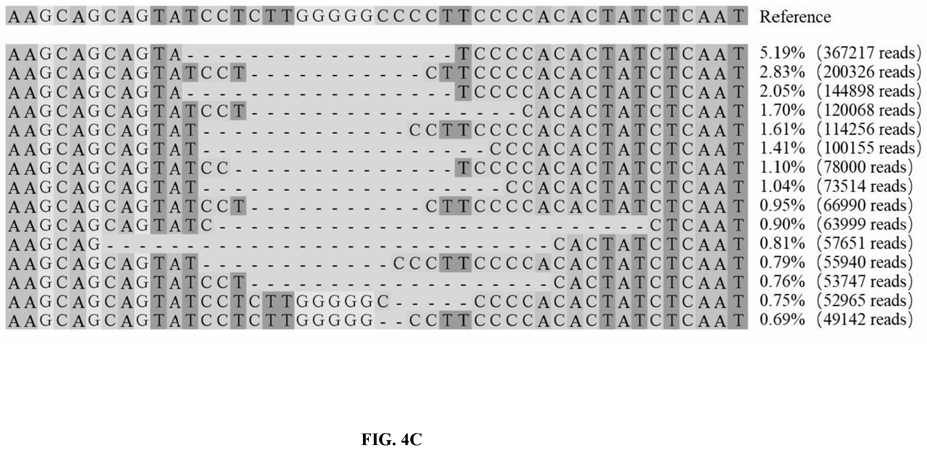

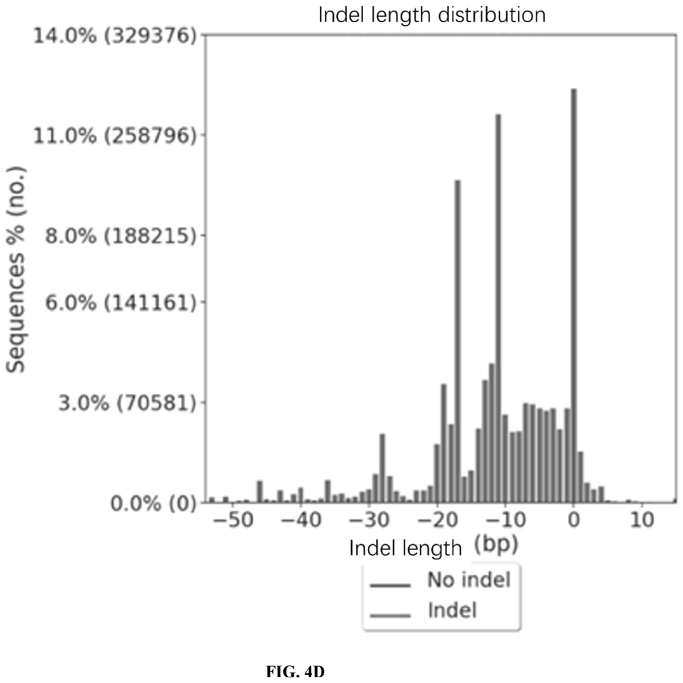

In the electroporation experiment in HSPCs, when the Cas : sgRNA molar ratio was set to be 1 : 1 to 1 : 3, the dose-dependent effects of different doses of RNP on editing efficiency were observed. The observations showed that the indel frequency on the target LRF-binding motif exceeded 85% when the dose of RNP was 180 pmol and the Cas : sgRNA molar ratio was 1 : 3 (FIG. 4A). NGS results demonstrate that high-efficiency editing is achieved in CD34+ HSPCs from both healthy donors and β-thalassemia (β⁰β⁰) patients (FIG. 4B); and the most frequent indels are deletions longer than 10 bp, generated by microhomology-mediated end joining (MMEJ) repair (FIGs. 4C and 4D).

### Example 4

### Evaluation of off-target effects of the selected Cas12b^{Max} and sgRNA-G1 in gene editing of hematopoietic stem cell

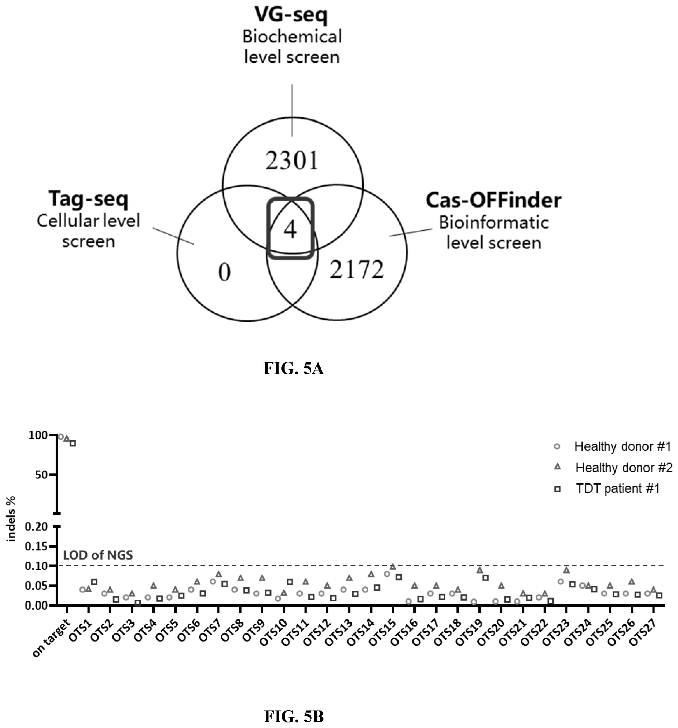

To evaluate the specificity of high-efficiency on-target editing by Cas12b^{Max}-RNP, two unbiased genome-wide methods were employed in this example: modified Site-seq and Tag-seq, which were used to screen non-target sequences susceptible to RNP cleavage in naked genomic DNA and the cellular context, respectively. Combined with bioinformatic prediction, a total of 4 potential off-target sites (OTSs) were found to overlap with the listed off-target sequences obtained by screening. These 4 potential off-target sites and 23 sites with high sequence similarities to the on-target sequence were validated by amplicon deep sequencing in highly edited CD34+ HSPCs, with the indel frequency of targeted editing being ≥ 90%. No RNP-mediated mutations were detected at any of the candidate OTSs, with indel frequencies below 0.1%, the detection limit of NGS (FIG. 5B). Taken together, these data demonstrate that the CD34+ HSPCs editing method provided in the present application is highly specific for the target sequence and does not have genetic or genomic toxicity detected.

### Off-target effect analysis method:

Site-seq experiment: Briefly, a purified genomic DNA was digested with Cas12b^{Max}-RNP in a reaction buffer. The digested gDNA was hybridized and ligated to a biotinylated, sticky-ended, Illumina-compatible adapter. The adapter-ligated gDNA was fragmented, subjected to end repair, and ligated to a second adapter. After purifying the double-adapter-ligated DNA, a two-step PCR was performed to construct a sequencing library. Sequencing data were analyzed using a Perl software package.

Tag-seq experiment: HEK293 cells were co-transfected with Cas12b^{Max}, a plasmid encoding a gRNA, and dsTag using lipo3000. The genomic DNA was purified, sheared to an average length of 300 bp, end-repaired, A-tailed and ligated to half-adapters, followed by incorporation of an 8-nt random molecular identifier. Two rounds of nested anchored PCR were used to construct target enrichment sequencing libraries. Sequencing data were analyzed by an open source Python software package.

To validate off-target effects in edited HSPCs by NGS, a two-step PCR procedure was used to amplify potential off-target sites. Amplicons were sequenced on the MiSeq sequencing system (Illumina) with 2×150 paired-end reads. Deep sequencing data were analyzed using the offline CRISPResso software.

### Example 5

### Induction of γ-globin and fetal hemoglobin by the selected Cas12b^{Max} and sgRNA-G1

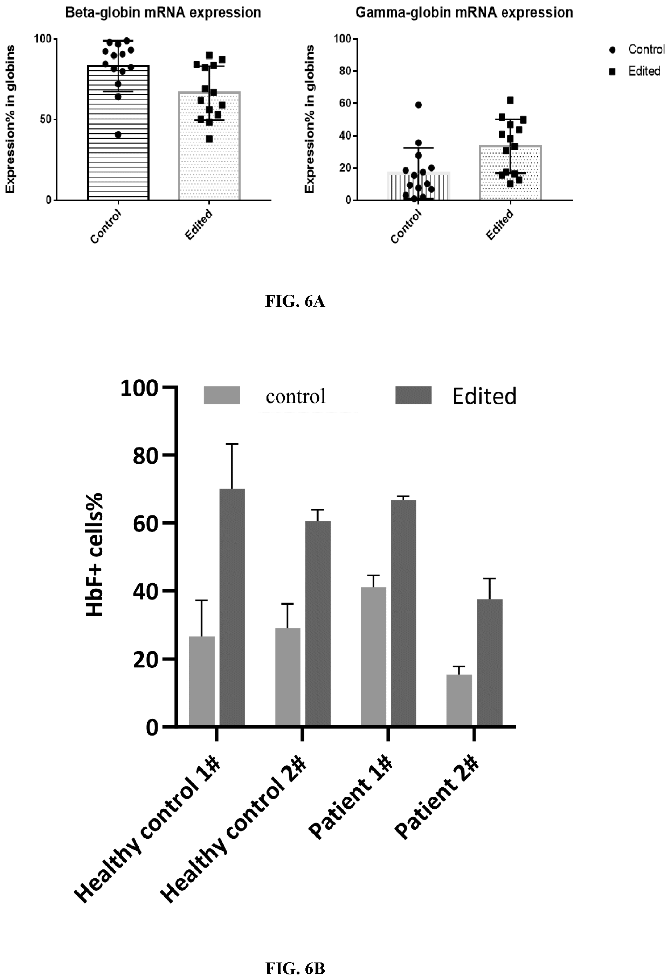

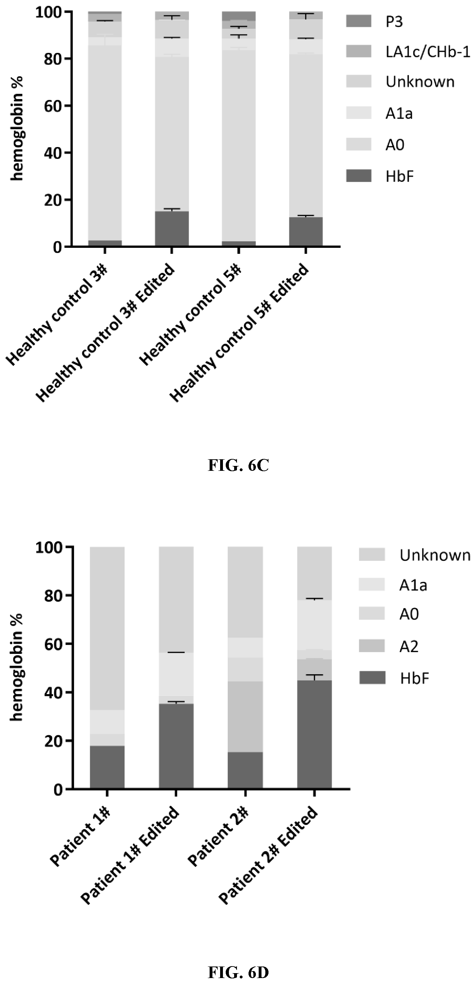

To test whether disruption of the LRF-binding motif in the promoter region of the HBG gene would result in significant γ-globin reactivation, HSPCs from healthy donors or β-thalassemia patients were induced to mature erythrocytes in vitro after electroporation with RNPs. The results demonstrate that, compared with erythroid cells derived from unedited HSPCs, erythroid progeny of HSPCs with a higher disruption rate of the LRF motif in the HBG promoter region exhibit an elevated γ-globin mRNA level and a decreased β-globin mRNA level (FIG. 6A). The expression of HbF protein was analyzed by high-performance liquid chromatography (HPLC) or low-magnification cell counting. In erythroid cells generated in vitro, disruption of the LRF binding motif in the HBG promoter region significantly increased the number of F cells (HbF/CD235a double-positive) (FIG. 6B). It is also observed from the results that the HbF protein content in total hemoglobin extracted from erythroid cells with disrupted LRF motif in the HBG promoter region is significantly increased compared with the healthy donor control group (FIGs. 6C and 6D).

### Example 6

### Other effects of Cas12b^{Max} and sgRNA-G1 gene editing on hematopoietic stem cells

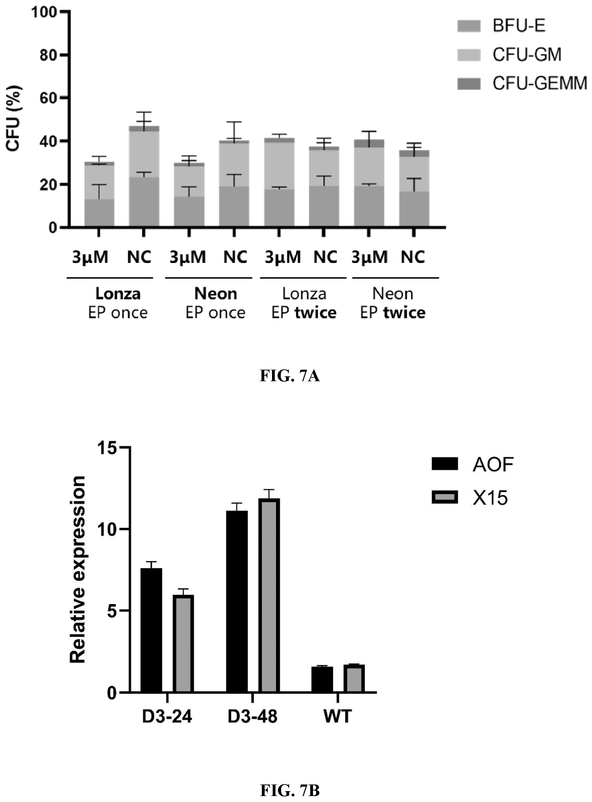

The multilineage differentiation potential of CD34+ HSPCs electroporated with Cas12bMax-RNP was analyzed by colony-forming unit assay. The results in FIG. 7A show that the gene-editing process has a minimal effect on the differentiation potential of hematopoietic stem cells. Additionally, the dynamics of P21 expression in CD34+ HSPCs following electroporation with Cas12bMax-RNP were analyzed by RT-PCR, as shown in FIG. 7B.

### Example 7

### In vivo efficacy of hematopoietic stem cells edited by Cas12b^{Max} and sgRNA-G1

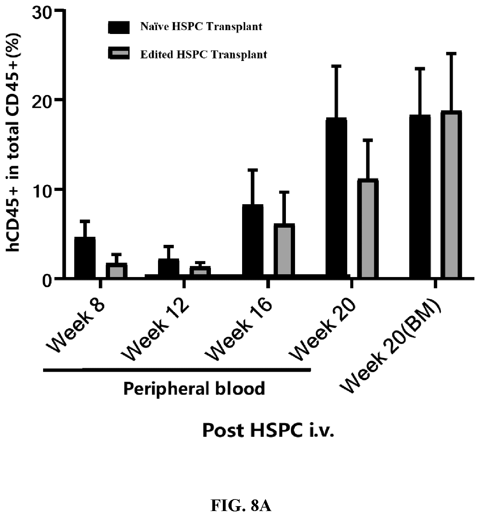

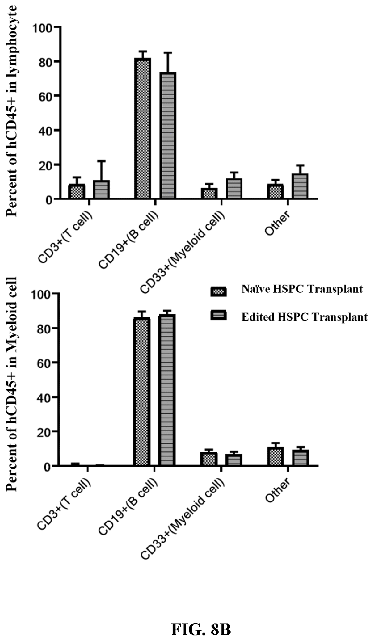

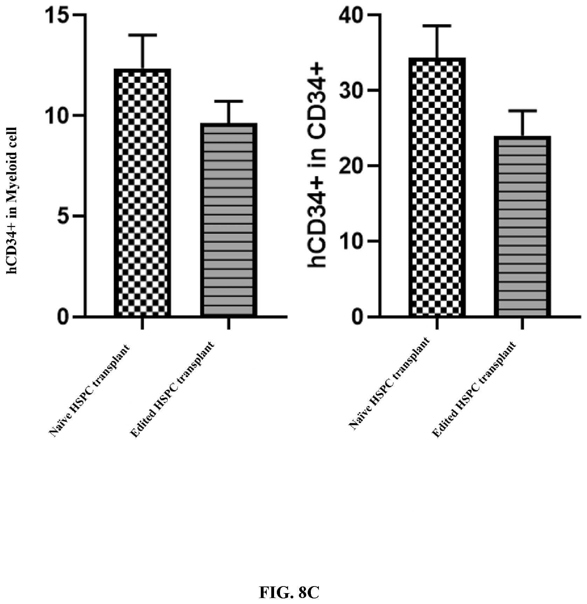

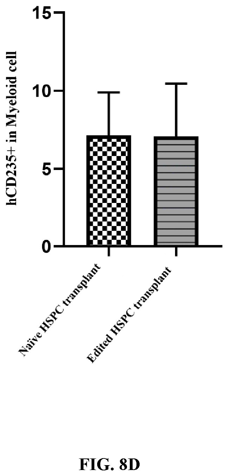

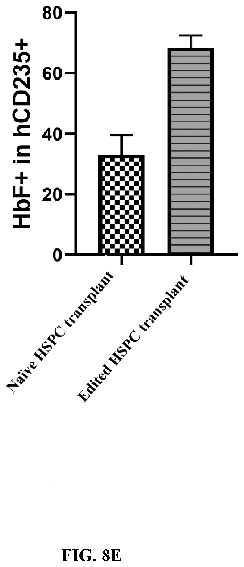

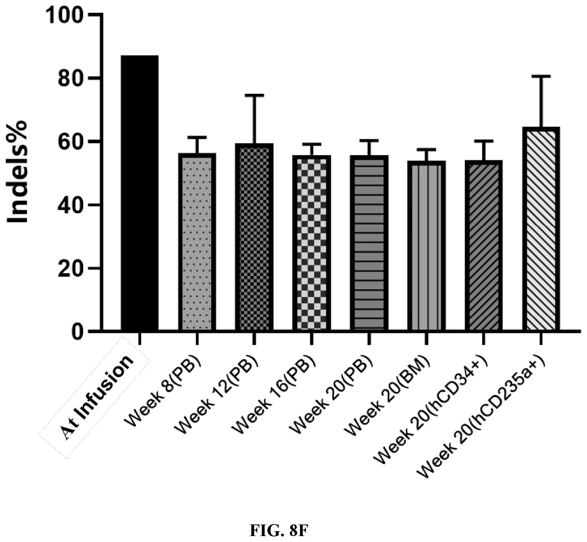

Edited CD34+ HSPCs from patients with β-thalassemia major (β⁰β⁰) were infused into immunodeficient NCG-X mice to evaluate the effect of the editing method of the present application on their engraftment capacity and hematopoietic differentiation potential. Immunodeficient NCG-X mice support hematopoietic stem cell transplantation without the need for myeloablative drug treatment. After transplantation, the hCD45+ cell chimerism rate in the peripheral circulation was lower in the RNP-electroporated hCD34+ HSPC transplant group as compared to the naïve CD34+ HSPC transplant group. At week 20, the levels of human lymphoid and myeloid (hCD45+) or erythroid (hCD235a+) reconstitution were similar in the bone marrow of subjects in both groups (FIGs. 8A, 8B and 8D). The number of hCD34+ cells in the bone marrow of recipient mice that received RNP-electroporated hCD34+ HSPCs was less (FIG. 8C). At week 20, substantial HbF+ cells were induced from hCD235+ cells in the bone marrow of mice infused with edited hCD34+ HSPCs (FIG. 8E). The frequency of targeted mutagenesis detected in human multilineage cells isolated from peripheral blood or bone marrow was reduced compared to that at the time of infusion of the edited HSPCs (FIG. 8F).

### Xenotransplantation of human CD34+ HSPCs:

NCG-X mice were purchased from GemPharatech Co., Ltd. Non-irradiated female NCG-X mice (4-5 weeks old) were injected via the tail vein with 0.5-0.6 × 10⁶ CD34+ HSPCs from healthy donors. CD34+ HSPCs used for transplantation experiments were cultured overnight in vitro after electroporation with RNPs. At 20 weeks post-transplantation, bone marrow was isolated for analysis of human cell chimerism and HbF+ cells. A secondary transplantation was performed by injecting hCD34+ HSPCs isolated from the bone marrow of primary subjects, via the tail vein. For flow cytometric analysis of bone marrow, bone marrow cells were first incubated with V450 mouse anti-human CD45 clone HI30 (560367, BD Biosciences), PE-eFluor 610 mCD45 monoclonal antibody (30-F11) (61-0451-82, Thermo Fisher), FITC anti-human CD235a antibody (349104, BioLegend), PE anti-human CD33 antibody (366608, BioLegend), APC anti-human CD19 antibody (302212, BioLegend), and fixable viability dye eFluor 780 (650865-14, Thermo Fisher) for live/dead staining. The percentage of human engraftment was calculated as: hCD45+ cells / (hCD45+ cells + mCD45+ cells) × 100. To assess the engraftment capacity of human CD34+ HSPCs, the CD34+ HSPCs were incubated with anti-human CD34 antibody (343512, Biolegend), anti-mouse CD34 antibody (303508, Biolegend), and dye eFluor 780 (650865-14, Thermo Fisher) for live/dead staining. Analysis of HbF+ cells was performed by isolating human CD235a+ cells from bone marrow using the hCD234a+ microbead kit (Miltenyi). The method for FACS analysis of HbF+ cells was the same as the method for measuring HbF+ cells with the flow cytometer as described above.

## Claims

1. A guide RNA (gRNA) molecule, wherein the gRNA molecule comprises a targeting domain complementary to a target sequence, and the target sequence is located within an HBG1 promoter region located between chr11: 5,248,269 and 5,249,857 of human genome hg19, or within an HBG2 promoter region located between chr11: 5,253,188 and 5,254,781 of human genome hg19.

2. The gRNA molecule according to claim 1, wherein the target sequence is located between about 210 bp upstream and about 100 bp upstream of transcription initiation sites of the HBG1 and HBG2.

3. The gRNA molecule according to any one of claims 1 and 2, comprising a tracr sequence and a crRNA sequence.

4. The gRNA molecule according to any one of claims 1-3, wherein the gRNA molecule is a dual guide RNA molecule.

5. The gRNA molecule according to any one of claims 1-3, wherein the gRNA molecule is a single guide RNA molecule (sgRNA).

6. The gRNA molecule according to any one of claims 1-5, wherein (1) a CRISPR/Cas system comprising the gRNA molecule is contacted with the target sequence, or (2) a CRISPR/Cas system comprising the gRNA molecule is introduced into a cell in which a gene comprising the target sequence is located, enabling a base insertion or deletion (indel) formed at or near the target sequence.

7. The gRNA molecule according to claim 6, wherein the CRISPR/Cas system comprises a Cas12b nuclease and/or a functionally active fragment thereof.

8. The gRNA molecule according to claim 6, wherein the CRISPR/Cas system comprises a Cas12i nuclease and/or a functionally active fragment thereof.

9. The gRNA molecule according to claim 3, wherein the tracr sequence comprises a nucleotide sequence set forth in SEQ ID NO: 60.

10. The gRNA molecule according to claim 3, wherein the crRNA sequence comprises a nucleotide sequence selected from any one of SEQ ID NOs: 61-66.

11. The gRNA molecule according to any one of claims 1-10, comprising a nucleotide sequence selected from any one of SEQ ID NOs: 12-59 and 67-78.

12. A nucleic acid molecule encoding the gRNA molecule according to any one of claims 1-11.

13. A vector comprising the nucleic acid molecule according to claim 12.

14. The vector according to claim 13, wherein the vector is selected from lentivirus vectors, adenovirus vectors, adeno-associated virus (AAV) vectors, herpes simplex virus (HSV) vectors, plasmids, minicircles, nanoplasmids and RNA vectors.

15. A ribonucleoprotein (RNP) complex, comprising the gRNA molecule according to any one of claims 1-11 and a nuclease of a CRISPR/Cas system.

16. The RNP complex according to claim 15, wherein the nuclease is a class II type V Cas enzyme.

17. The RNP complex according to claim 16, wherein the nuclease is a Cas12b nuclease.

18. The RNP complex according to claim 17, wherein the Cas12b nuclease comprises a variant thereof, an ortholog thereof and/or a functionally active fragment thereof.

19. The RNP complex according to claim 17 or 18, wherein the Cas12b nuclease is a Cas12b nuclease from *Alicyclobacillus acidiphilus* (AaCas12b).

20. The RNP complex according to claim 19, wherein the AaCas12b nuclease comprises one or more of the following mutations relative to wild-type AaCas12b nuclease:
(1) a substitution of one or more amino acid residues in the wild-type AaCas12b nuclease that interact with a protospacer adjacent motif (PAM) with a positively charged amino acid residue(s);
(2) a substitution of one or more amino acid residues involved in opening a DNA double strand in the wild-type AaCas12b nuclease with an amino acid residue(s) having an aromatic ring; and/or
(3) a substitution of one or more amino acid residues in RuvC domain of the wild-type AaCas12b nuclease that interact with a single-stranded DNA substrate with a positively charged amino acid residue(s) or a hydrophobic amino acid residue(s),
wherein the amino acid sequence of the wild-type AaCas12b nuclease is as set forth in SEQ ID NO: 91.

21. The RNP complex according to claim 20, wherein the one or more amino acid residues that interact with PAM are located at one or more of positions 116, 123, 130, 132, 144, 145, 153, 173, 222, 395, 400 and 475; and wherein the amino acid residues are numbered according to SEQ ID NO: 91.

22. The RNP complex according to claim 20 or 21, wherein the substitution of one or more amino acid residues in the wild-type AaCas12b nuclease that interact with PAM with a positively charged amino acid residue(s) is one or more of substitutions D116R and E475R; and wherein the amino acid residues are numbered according to SEQ ID NO: 91.

23. The RNP complex according to claim 20, wherein the one or more amino acid residues involved in opening the DNA double strand are located at one or more of positions 118 and 119; and wherein the amino acid residues are numbered according to SEQ ID NO: 91.

24. The RNP complex according to claim 20 or 23, wherein the substitution of one or more amino acid residues involved in opening the DNA double strand in the wild-type AaCas12b nuclease with an amino acid residue(s) having an aromatic ring is substitution Q119Y, Q119F or Q119W; and wherein the amino acid residues are numbered according to SEQ ID NO: 91.

25. The RNP complex according to claim 20, wherein the one or more amino acid residues in RuvC domain that interact with a single-stranded DNA substrate are located at one or more positions 300, 301, 304, 329, 636, 639, 647, 682, 757, 758, 761, 764, 768, 852, 854, 856, 857, 858, 860, 862, 863, 865, 866, 867, 869, 938, 956, 957, 958, 994, 1093 and 1097; and wherein the amino acid residues are numbered according to SEQ ID NO: 91.

26. The RNP complex according to claim 20 or 25, wherein the substitution of one or more amino acid residues in RuvC domain of the wild-type AaCas12b nuclease that interact with a single-stranded DNA substrate is one or more of substitutions E636R, Q639R, T647R, Q682R, I757R, E758R, E761R, Q854R, N857K, D858R, I994R, Q1093R and W1097R; and wherein the amino acid residues are numbered according to SEQ ID NO: 91.

27. The RNP complex according to any one of claims 17-26, wherein the gRNA molecule comprises a nucleotide sequence set forth in any one of SEQ ID NOs: 12-59.

28. The RNP complex according to claim 16, wherein the nuclease is a Cas12i nuclease.

29. The RNP complex according to claim 28, wherein the Cas12i nuclease comprises a variant thereof, an ortholog thereof and/or a functionally active fragment thereof.

30. The RNP complex according to claim 28 or 29, wherein the Cas12i nuclease comprises one or more of the following mutations relative to wild-type Cas12i2 nuclease:
(1) a substitution of one or more amino acids in the wild-type Cas12i2 nuclease that interact with PAM with a positively charged amino acid(s);
(2) a substitution of one or more amino acids involved in opening a DNA double strand in the wild-type Cas12i2 nuclease with an amino acid(s) having an aromatic ring;
(3) a substitution of one or more amino acids in RuvC domain of the wild-type Cas12i2 nuclease that interact with a single-stranded DNA substrate with a positively charged amino acid(s); and/or
(4) a substitution of one or more amino acids in the wild-type Cas12i2 nuclease that interact with a DNA-RNA duplex with a positively charged amino acid(s),
wherein the amino acid sequence of the wild-type Cas12i2 nuclease is as set forth in SEQ ID NO: 92.

31. The RNP complex according to claim 30, wherein the one or more amino acids that interact with PAM are located at one or more of positions 176, 238, 447 and/or 563; and wherein the positions are numbered according to SEQ ID NO: 92.

32. The RNP complex according to claim 30, wherein the positively charged amino acid in (1) is R or K.

33. The RNP complex according to any one of claims 28-32, wherein the Cas12i nuclease comprises any one of the following mutations or combinations of mutations: (1) E563R; (2) E176R, T447R, E176R and E563R; (3) K238R and E563R; (4) E176R, K238R and T447R; (5) E176R, K238R and E563R; (6) E176R, T447R and E563R; and/or (7) E176R, K238R, T447R and E563R; and wherein the amino acid residues are numbered according to SEQ ID NO: 92.

34. The RNP complex according to claim 30, wherein the one or more amino acids involved in opening a DNA double strand are located at one or more of positions 163 and/or 164; and wherein the positions are numbered according to SEQ ID NO: 92.

35. The RNP complex according to claim 30, wherein the amino acid having an aromatic ring in (2) is F, Y or W.

36. The RNP complex according to claim 34 or 35, wherein the substitution of one or more amino acids involved in opening a DNA double strand with an amino acid(s) having an aromatic ring refers to Q163F, Q163Y, Q163W and/or N164F or N164Y; and wherein the amino acid residues are numbered according to SEQ ID NO: 92.

37. The RNP complex according to claim 30, wherein the one or more amino acids in RuvC domain that interact with a single-stranded DNA substrate are located at one or more of positions 323, 362, 425, 925, 926, 391, 424 and/or 929; and wherein the positions are numbered according to SEQ ID NO: 92.

38. The RNP complex according to claim 30, wherein the positively charged amino acid in (3) is R or K.

39. The RNP complex according to claim 37 or 38, wherein the Cas12i nuclease comprises any one of the following mutations or combinations of mutations: (1) E323R; (2) D362R; (3) Q425R; (4) N925R; (5) I926R; (6) E323R and D362R; (7) E323R and Q425R; (8) E323R and I926R; (9) Q425R and I926R; (10) D362R and I926R; (11) N925R and I926R; (12) E323R, D362R and Q425R; (13) E323R, D362R and I926R; (14) E323R, Q425R and I926R; (15) D362R, N925R and I926R; and/or (16) E323R, D362R, Q425R and I926R; and wherein the amino acid residues are numbered according to SEQ ID NO: 92.

40. The RNP complex according to claim 30, wherein the one or more amino acids that interact with a DNA-RNA duplex are located at one or more of positions 116, 117, 159, 161, 319, 343 and/or 958; and wherein the positions are numbered according to SEQ ID NO: 92.

41. The RNP complex according to claim 30, wherein the positively charged amino acid in (4) is R or K.

42. The RNP complex according to claim 40 or 41, wherein the Cas12i nuclease comprises any one of the following mutations or combinations of mutations: G116R, E117R, T159R, S161R, E319R, E343R and/or D958R; and wherein the amino acid residues are numbered according to SEQ ID NO: 92.

43. The RNP complex according to any one of claims 30-42, wherein the Cas12i nuclease further comprises one or more mutations in a flexible region that are capable of imparting increased flexibility in the flexible region of the Cas12i nuclease as compared to the wild-type Cas12i2 nuclease.

44. The RNP complex according to claim 43, wherein the flexible region is selected from the group consisting of regions corresponding to amino acid residues 228-232, amino acid residues 439-443, amino acid residues 478-482, amino acid residues 500-504, amino acid residues 775-779, and amino acid residues 925-929; and wherein the amino acid residues are numbered according to SEQ ID NO: 92.

45. The RNP complex according to claim 43 or 44, wherein the one or more mutations in a flexible region comprise an insertion of one or more G residues in the flexible region.

46. The RNP complex according to claim 45, wherein the one or more G residues are inserted N-terminally to a flexible amino acid residue in the flexible region, and wherein the flexible amino acid residue is selected from the group consisting of G, S, N, D, H, M, T, E, Q, K, R, A and P.

47. The RNP complex according to claim 43 or 44, wherein the one or more mutations in a flexible region comprises a substitution of a hydrophobic amino acid residue in the flexible region with a G residue, and wherein the hydrophobic amino acid residue is selected from the group consisting of L, I, V, C, Y, F and W.

48. The RNP complex according to any one of claims 43-47, wherein the one or more mutations in a flexible region are: (1) I926G; and/or (2) 439G or 439GG, and wherein the amino acid residues are numbered according to SEQ ID NO: 92.

49. The RNP complex according to any one of claims 43-48, wherein the Cas12i nuclease comprises mutations E176R, K238R, T447R, E563R, N164Y, E323R and D362R; and wherein the amino acid residues are numbered according to SEQ ID NO: 92.

50. The RNP complex according to any one of claims 28-49, wherein the gRNA molecule comprises a nucleotide sequence set forth in any one of SEQ ID NOs: 67-78.

51. The RNP complex according to any one of claims 15-49, wherein the N-terminus and/or C-terminus of the nuclease is linked to one or more nuclear localization signals.

52. The RNP complex according to claim 51, wherein the nuclear localization signal is linked to a His tag.

53. The RNP complex according to any one of claims 15-52, wherein the nuclease comprises an amino acid sequence selected from any one of SEQ ID NOs: 1-11.

54. A composition comprising:
(1) one or more gRNA molecules according to any one of claims 1-11, and a nuclease of a CRISPR/Cas system;
(2) a nucleic acid encoding one or more gRNA molecules according to any one of claims 1-11, and a nuclease of a CRISPR/Cas system;
(3) one or more gRNA molecules according to any one of claims 1-11, and a nucleic acid encoding a nuclease of a CRISPR/Cas system; or
(4) a nucleic acid encoding one or more gRNA molecules according to any one of claims 1-11, and a nucleic acid encoding a nuclease of a CRISPR/Cas system.

55. The composition according to claim 54, wherein the one or more gRNA molecules and the nuclease of a CRISPR/Cas system in (1) are present in a ribonucleoprotein (RNP) complex.

56. The composition according to claim 55, wherein the nuclease is a class II type V Cas enzyme.

57. The composition according to claim 56, wherein the nuclease is a Cas12b nuclease.

58. The composition according to claim 57, wherein the Cas12b nuclease comprises a variant thereof, an ortholog thereof and/or a functionally active fragment thereof.

59. The composition according to claim 57 or 58, wherein the Cas12b nuclease is a Cas12b nuclease from *Alicyclobacillus acidiphilus* (AaCas12b).

60. The composition according to claim 59, wherein the AaCas12b comprises one or more of the following mutations relative to wild-type AaCas12b nuclease:
(1) a substitution of one or more amino acid residues in the wild-type AaCas12b nuclease that interact with a protospacer adjacent motif (PAM) with a positively charged amino acid residue(s);
(2) a substitution of one or more amino acid residues involved in opening a DNA double strand in the wild-type AaCas12b nuclease with an amino acid residue(s) having an aromatic ring; and/or
(3) a substitution of one or more amino acid residues in RuvC domain of the wild-type AaCas12b nuclease that interact with a single-stranded DNA substrate with a positively charged amino acid residue(s) or a hydrophobic amino acid residue(s),
wherein the amino acid sequence of the wild-type AaCas12b nuclease is as set forth in SEQ ID NO: 91.

61. The composition according to claim 60, wherein the one or more amino acid residues that interact with PAM are located at one or more of positions 116, 123, 130, 132, 144, 145, 153, 173, 222, 395, 400 and 475; and wherein the amino acid residues are numbered according to SEQ ID NO: 91.

62. The composition according to claim 60 or 61, wherein the substitution of one or more amino acid residues in the wild-type AaCas12b nuclease that interact with PAM with a positively charged amino acid residue(s) is one or more of substitutions D116R and E475R; and wherein the amino acid residues are numbered according to SEQ ID NO: 91.

63. The composition according to claim 60, wherein the one or more amino acid residues involved in opening the DNA double strand are located at one or more of positions 118 and 119; and wherein the amino acid residues are numbered according to SEQ ID NO: 91.

64. The composition according to claim 60 or 63, wherein the substitution of one or more amino acid residues involved in opening the DNA double strand in the wild-type AaCas12b nuclease with an amino acid residue(s) having an aromatic ring is substitution Q119Y, Q119F or Q119W; and wherein the amino acid residues are numbered according to SEQ ID NO: 91.

65. The composition according to claim 60, wherein the one or more amino acid residues in RuvC domain that interact with a single-stranded DNA substrate are located at one or more positions 300, 301, 304, 329, 636, 639, 647, 682, 757, 758, 761, 764, 768, 852, 854, 856, 857, 858, 860, 862, 863, 865, 866, 867, 869, 938, 956, 957, 958, 994, 1093 and 1097; and wherein the amino acid residues are numbered according to SEQ ID NO: 91.

66. The composition according to claim 60 or 65, wherein the substitution of one or more amino acid residues in RuvC domain of the wild-type AaCas12b nuclease that interact with a single-stranded DNA substrate is one or more of substitutions E636R, Q639R, T647R, Q682R, I757R, E758R, E761R, Q854R, N857K, D858R, I994R, Q1093R and W1097R; and wherein the amino acid residues are numbered according to SEQ ID NO: 91.

67. The composition according to any one of claims 57-66, wherein the gRNA molecule comprises a nucleotide sequence set forth in any one of SEQ ID NOs: 12-59.

68. The composition according to claim 56, wherein the nuclease is a Cas12i nuclease.

69. The composition according to claim 68, wherein the Cas12i nuclease comprises a variant thereof, an ortholog thereof and/or a functionally active fragment thereof.

70. The composition according to claim 68 or 69, wherein the Cas12i nuclease comprises one or more of the following mutations relative to wild-type Cas12i2 nuclease:
(1) a substitution of one or more amino acids in the wild-type Cas12i2 nuclease that interact with PAM with a positively charged amino acid(s);
(2) a substitution of one or more amino acids involved in opening a DNA double strand in the wild-type Cas12i2 nuclease with an amino acid(s) having an aromatic ring;
(3) a substitution of one or more amino acids in RuvC domain of the wild-type Cas12i2 nuclease that interact with a single-stranded DNA substrate with a positively charged amino acid(s); and/or
(4) a substitution of one or more amino acids in the wild-type Cas12i2 nuclease that interact with a DNA-RNA duplex with a positively charged amino acid(s),
wherein the amino acid sequence of the wild-type Cas12i2 nuclease is as set forth in SEQ ID NO: 92.

71. The composition according to claim 70, wherein the one or more amino acids that interact with PAM are located at one or more of positions 176, 238, 447 and/or 563; and wherein the positions are numbered according to SEQ ID NO: 92.

72. The composition according to claim 70, wherein the positively charged amino acid in (1) is R or K.

73. The composition according to claim 71 or 72, wherein the Cas12i nuclease comprises any one of the following mutations or combinations of mutations: (1) E563R; (2) E176R, T447R, E176R and E563R; (3) K238R and E563R; (4) E176R, K238R and T447R; (5) E176R, K238R and E563R; (6) E176R, T447R and E563R; and/or (7) E176R, K238R, T447R and E563R; and wherein the amino acid residues are numbered according to SEQ ID NO: 92.

74. The composition according to claim 70, wherein the one or more amino acids involved in opening a DNA double strand are located at one or more of positions 163 and/or 164; and wherein the positions are numbered according to SEQ ID NO: 92.

75. The composition according to claim 70, wherein the amino acid having an aromatic ring in (2) is F, Y or W.

76. The composition according to claim 74 or 75, wherein the substitution of one or more amino acids involved in opening a DNA double strand with an amino acid(s) having an aromatic ring refers to Q163F, Q163Y, Q163W and/or N164F or N164Y; and wherein the amino acid residues are numbered according to SEQ ID NO: 92.

77. The composition according to claim 70, wherein the one or more amino acids in RuvC domain that interact with a single-stranded DNA substrate are located at one or more of positions 323, 362, 425, 925, 926, 391, 424 and/or 929; and wherein the positions are numbered according to SEQ ID NO: 92.

78. The composition according to claim 70, wherein the positively charged amino acid in (3) is R or K.

79. The composition according to claim 77 or 78, wherein the Cas12i nuclease comprises any one of the following mutations or combinations of mutations: (1) E323R; (2) D362R; (3) Q425R; (4) N925R; (5) I926R; (6) E323R and D362R; (7) E323R and Q425R; (8) E323R and I926R; (9) Q425R and I926R; (10) D362R and I926R; (11) N925R and I926R; (12) E323R, D362R and Q425R; (13) E323R, D362R and I926R; (14) E323R, Q425R and I926R; (15) D362R, N925R and I926R; and/or (16) E323R, D362R, Q425R and I926R; and wherein the amino acid residues are numbered according to SEQ ID NO: 92.

80. The composition according to claim 70, wherein the one or more amino acids that interact with a DNA-RNA duplex are located at one or more of positions 116, 117, 159, 161, 319, 343 and/or 958; and wherein the positions are numbered according to SEQ ID NO: 92.

81. The composition according to claim 70, wherein the positively charged amino acid in (4) is R or K.

82. The composition according to claim 80 or 81, wherein the Cas12i nuclease comprises any one of the following mutations or combinations of mutations: G116R, E117R, T159R, S161R, E319R, E343R and/or D958R; and wherein the amino acid residues are numbered according to SEQ ID NO: 92.

83. The composition according to any one of claims 70-82, wherein the Cas12i nuclease further comprises one or more mutations in a flexible region that are capable of imparting increased flexibility in the flexible region of the Cas12i nuclease as compared to the wild-type Cas12i2 nuclease.

84. The composition according to claim 83, wherein the flexible region is selected from the group consisting of regions corresponding to amino acid residues 228-232, amino acid residues 439-443, amino acid residues 478-482, amino acid residues 500-504, amino acid residues 775-779, and amino acid residues 925-929; and wherein the amino acid residues are numbered according to SEQ ID NO: 92.

85. The composition according to claim 83 or 84, wherein the one or more mutations in a flexible region comprise an insertion of one or more G residues in the flexible region.

86. The composition according to claim 85, wherein the one or more G residues are inserted N-terminally to a flexible amino acid residue in the flexible region, and wherein the flexible amino acid residue is selected from the group consisting of G, S, N, D, H, M, T, E, Q, K, R, A and P.

87. The composition according to claim 85 or 86, wherein the one or more mutations in a flexible region comprises a substitution of a hydrophobic amino acid residue in the flexible region with a G residue, and wherein the hydrophobic amino acid residue is selected from the group consisting of L, I, V, C, Y, F and W.

88. The composition according to any one of claims 83-87, wherein the one or more mutations in a flexible region are: (1) I926G; and/or (2) 439G or 439GG, and wherein the amino acid residues are numbered according to SEQ ID NO: 92.

89. The composition according to any one of claims 68-88, wherein the Cas12i nuclease comprises mutations E176R, K238R, T447R, E563R, N164Y, E323R and D362R; and wherein the amino acid residues are numbered according to SEQ ID NO: 92.

90. The composition according to any one of claims 68-89, wherein the gRNA molecule comprises a nucleotide sequence set forth in any one of SEQ ID NOs: 67-78.

91. The composition according to any one of claims 54-90, wherein the N-terminus and/or C-terminus of the nuclease is linked to one or more nuclear localization signals.

92. The composition according to claim 91, wherein the nuclear localization signal is linked to a His tag.

93. The composition according to any one of claims 54-92, wherein the nuclease comprises an amino acid sequence selected from any one of SEQ ID NOs: 1-11.

94. The composition according to any one of claims 54-93, wherein the composition further optionally comprises a pharmaceutically acceptable carrier.

95. A cell, comprising the gRNA molecule according to any one of claims 1-11, the nucleic acid molecule according to claim 12, the vector according to any one of claims 13 and 14, the ribonucleoprotein (RNP) complex according to any one of claims 15-53, and/or the composition according to any one of claims 54-94.

96. A cell population or progeny thereof, comprising a gene product produced by administration of the ribonucleoprotein (RNP) complex according to any one of claims 15-53, the composition according to any one of claims 54-94, or the cell according to claim 95.

97. The cell population or progeny thereof according to claim 96, wherein the gene product comprises an HBG gene having a modification.

98. The cell population or progeny thereof according to claim 97, wherein the modification is a base insertion or deletion (indel) at or near a target sequence; wherein the target sequence is located within an HBG1 promoter region between chr11: 5,248,269 and 5,249,857 of human genome hg19, or within an HBG2 promoter region between chr11: 5,253,188 and 5,254,781 of human genome hg19.

99. The cell population or progeny thereof according to claim 98, wherein the target sequence is located between about 210 bp upstream and about 100 bp upstream of transcription initiation sites of the HBG1 and HBG2.

100. The cell population or progeny thereof according to any one of claims 96-99, wherein the cell population is capable of differentiating into differentiated cells of an erythroid cell lineage, and the differentiated cells have an increased level of fetal hemoglobin expression as compared to a cell population in which the HBG gene is not modified.

101. A kit, comprising the gRNA molecule according to any one of claims 1-11, the nucleic acid molecule according to claim 12, the vector according to any one of claims 13 and 14, the ribonucleoprotein (RNP) complex according to any one of claims 15-53, the composition according to any one of claims 54-94, the cell according to claim 95, and/or the cell population or progeny thereof according to any one of claims 96-100.

102. A method for regulating fetal hemoglobin (HbF) expression in a cell, cell population or progeny thereof, comprising administering the ribonucleoprotein (RNP) complex according to any one of claims 15-53, the composition according to any one of claims 54-94, the cell according to claim 95, the cell population or progeny thereof according to any one of claims 96-100, and/or the kit according to claim 101.

103. The method according to claim 102, wherein the cell or cell population is a hematopoietic stem/progenitor cell (HSPC).

104. The method according to claim 103, wherein the cell or cell population is CD34+ HSPC.

105. A method for treating, preventing or alleviating a hemoglobinopathy and/or related condition thereof, comprising administering to a subject in need thereof an effective amount of the ribonucleoprotein (RNP) complex according to any one of claims 15-53, the composition according to any one of claims 54-94, the cell according to claim 95, the cell population or progeny thereof according to any one of claims 96-100, and/or the kit according to claim 101.

106. The method according to claim 105, wherein the hemoglobinopathy and/or related condition thereof is selected from: sickle cell disease, sickle cell anemia, hemoglobin C disease, hemoglobin C trait, hemoglobin S/C disease, hemoglobin D disease, hemoglobin E disease, thalassemia, a hemoglobinopathy with decreased oxygen affinity, and an unstable hemoglobinopathy.
